# EUROPEAN PATENT SPECIFICATION

(11) **EP 1 004 241 B1**
(45) Date of publication and mention of the grant of the patent: **28.04.2004**
(21) Application number: 99309154.5
(22) Date of filing: 17.11.1999
(51) Int. Cl.: A01N 47/38, A01N 43/824, C07D 271/10, C07D 271/12, C07D 413/04, C07D 417/04

(54) **2-Aryl-Delta2-1,3,4- (oxa and thia)diazoline insecticidal and acaricidal agents**
2-Aryl-Delta 2-1,3,4-(oxa oder thia)Diazoline enthaltende Insektizide und akarizide Mittel
Agents insecticides et acaricides contenant des 2-aryl-delta 2-1,3,4-(oxa ou thia)diazolines

(30) Priority: 23.11.1998 US 197969
(43) Date of publication of application: 31.05.2000
(73) Proprietor: BASF AKTIENGESELLSCHAFT, 67056 Ludwigshafen (DE)
(72) Inventor: Takasugi, James Jan, Lawrenceville, New Jersey 08648 (US); Buckwalter, Brian Lee, Yardley, Pennsylvania 19067 (US)
(74) Representative: Köster, Reinhold, Dr.

(56) References cited:
- GB-A- 2 258 462
- US-A- 4 140 787
- DATABASE CROSSFIRE Beilstein Institut für Literatur der organischen Chemie XP002131018 & J. CHEM. SOC. PERKIN TRANS. 1, 1986, pages 1499-1506,
- DATABASE CROSSFIRE Beilstein Institut für Literatur der organischen Chemie XP002131019 & J. CHEM. SOC. PERKIN TRANS. 1, no. 9, 1982, pages 1993-1998,
- DATABASE CROSSFIRE Beilstein Institut für Literatur der organischen Chemie XP002131020 & J. ORG. CHEM. USSR, vol. 20, 1984, pages 152-162,
- DATABASE CROSSFIRE Beilstein Institut für Literatur der organischen Chemie XP002131021 & J. PHARM. CHIM., vol. 8, no. 22, 1935, page 289, 296
- DATABASE CROSSFIRE Beilstein Institut für Literatur der organischen Chemie XP002131022 & ARK. KEMI, vol. 9, 1956, page 47, 57
- DATABASE CROSSFIRE Beilstein Institut für Literatur der organischen Chemie XP002131023 & ACTA CHEM. SCAND., vol. 14, 1960, pages 789-796,
- DATABASE CROSSFIRE Beilstein Institut für Literatur der organischen Chemie XP002131024 & ARG. MASS SPECTROM., vol. 9, 1974, page 181, 182
- DATABASE CROSSFIRE Beilstein Institut für Literatur der organischen Chemie XP002131025 & BULL. SOC. CHIM. BELG., vol. 46, 1937, page 231, 239
- DATABASE CROSSFIRE Beilstein Institut für Literatur der organischen Chemie XP002131026 & BULL. SOC. CHIM. BELG., vol. 43, 1934, page 206, 207, 208, 209
- DATABASE CROSSFIRE Beilstein Institut für Literatur der organischen Chemie XP002131027 & BULL. SOC. CHIM. BELG., vol. 43, 1934, page 261, 267
- CHEMICAL ABSTRACTS, vol. 117, no. 11, 14 September 1992 Columbus, Ohio, US; abstract no. 111534f, SHABAN M A E ET AL: "Synthesis and biological activities of some 1,3,4-oxadiazoles and bis(1,3,4-oxadiazoles)" page 859; XP002131017 & ALEXANDRIA J. PHARM. SCI., vol. 6, no. 1, 1992, pages 17-21,

## Description

Insect and acarid pests destroy growing and harvested crops. In the United States, agronomic crops must compete with thousands of those pests. In particular, tobacco budworms and southern armyworms are especially devastating to crops.

Tobaccco budworms cause tremendous economic losses in agronomic crops. In particular, budworms devastate cotton crops by feeding on green bolls. Control of budworms is complicated by their resistance to many common insecticides, including organophosphates, carbamates and pyrethroids.

In spite of the commercial insecticides and acaricides available today, damage to crops, both growing and harvested, caused by insect and acarid pests still occurs. Accordingly, there is ongoing research to create new and more effective insecticidal and acaricidal agents.

Certain N-carbamoyl-3-carboxyaryl-heterocyclic and hydrazinecarboximidamidohydrazone compounds which are useful as herbicidal agents are described in US 5,670,456. However, that patent does not describe any insecticidal or acaricidal activity.

Certain cyclic 1,3,4-oxadiazoline compounds are described by D. Kochetov et al in Ukrainskii Khimicheskii Zhurnal, 57(2), pp. 215-217 (1991). 2-aryl-thiadiazoline compounds of the formula I are already disclosed in Org. Mass Spectrom. 9, pp. 181/182 (1974), Acta Chem. Scand. 14, pp. 789-796 (1960), Ark. Kemi 7, pp. 517/520 (1955), Ark. Kemi 9, pp. 47/57 (1956), Zh. Org. Khim 20 (1), pp. 169-180 (1984), J. Chem. Soc. Perkin Trans. 1, pp. 1499-1506 (1986), J. Chem. Soc. Perkin Trans. 1, no. 9, pp. 1993-1998 (1982), J. Pharm. Chim., vol. 8, no. 22, pp. 289/296 (1935), Bull. Soc. Chim. Belg. 46, pp. 231/239 (1937), Bull. Soc. Chim. Belg. 43, pp. 206-209, 261 & 267 (1934). However, D. Kochetov et al and the 10 references cited thereafter do not disclose any utility for their cyclic 1,3,4-oxa- and thiadiazoline compounds.

It was, therefore, an object of the present invention to provide compounds which are useful for the control of insect and acarid pests.

It was also an object of the present invention to provide a method for the control of insect and acarid pests.

It was a further object of this invention to provide a method for the protection of growing and harvested crops from damage caused by insect and acarid attack and infestation.

These and other objects of the present invention will become more apparent from the description thereof set forth below.

The present invention comprises 2-aryl-Δ²-1,3,4-(oxa and thia)diazoline compounds which are useful for the control of insect and acarid pests. Those compounds are also useful for protecting plants from damage caused by insect and acarid attack and infestation.

The pesticidal 2-aryl-Δ²-1,3,4-(oxa and thia)diazoline compounds of the present invention have the structural formula I 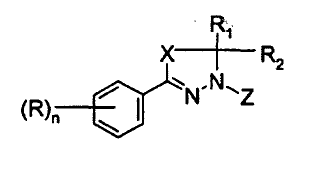 wherein
X is O or S(O)ₘ;
Z is 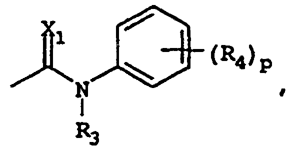 C(X₁)R₅, C₁-C₆alkyl, C₁-C₆haloalkyl, benzyl optionally substituted on the phenyl ring with any combination of from one to three halogen, C₁-C₆alkyl, C₁-C₆haloalkyl, C₁-C₆alkoxy,
C₁-C₆haloalkoxy, C₁-C₆alkylthio or C₁-C₆haloalkylthio groups, or
phenyl optionally substituted with any combination of from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups, provided that when X is O, Z is 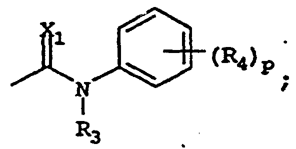 n and p are each independently 0, 1, 2 or 3;
X₁ is O or S;
R and R₄ are each independently halogen, C₁-C₆alkyl, C₁-C₆haloalkyl, OR₆, S(O)_{q}R₇, nitro, cyano, NR₈R₉, CO₂R₁₀, C(O)R₁₁ or
phenyl optionally substituted with any combination of from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups, or
two adjacent R groups or R₄ groups may be taken together to form a ring wherein RR or R₄R₄ is represented by: -OCH₂O-, -OCF₂O- or -CH=CH-CH=CH-;
R₆ and R₇ are each independently hydrogen, C₁-C₆alkyl, C₁-C₆haloalkyl or
phenyl optionally substituted with any combination of from one to three halogen, C₁-C₆alkyl, C₁-C₆haloalkyl, C₁-C₆alkoxy, C₁-C₄haloalkoxy, C₁-C₆alkylthio or C₁-C₆haloalkylthio groups;
R₈, R₉, R₁₃ and R₁₄ are each independently hydrogen, C₁-C₆alkyl, C₁-C₆alkylcarbonyl or
phenyl optionally substituted with any combination of from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups;
R₁₀ and R₁₁ are each independently hydrogen, C₁-C₆alkyl or C₁-C₆haloalkyl;
R₁ and R₂ are each independently hydrogen, C₃-C₇cycloalkyl,
C₁-C₆haloalkyl, C₃-C₆alkenyl, C₃-C₆haloalkenyl, C₃-C₆alkynyl, C₃-C₆haloalkynyl, C₂-C₆alkoxyalkyl, (CH₂)ᵥC(O)R₁₂,
C₁-C₆alkyl optionally substituted with one phenoxy or phenyl group wherein the phenyl ring of each group is independently, optionally substituted with from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups,
phenyl optionally substituted with from one to three halogen, C₁-C₆alkyl, C₁-C₆haloalkyl, C₁C₆alkoxy, C₁-C₆haloalkoxy, C₁-C₆alkylthio or C₁-C₆haloalkylthio groups, or
a 5- or 6-membered heteroaromatic ring optionally substituted with any combination of from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups, and
when R₁ and R₂ are taken together with the atom to which they are attached they may form a C₃-C₆cycloalkyl ring wherein R₁R₂ is represented by: -(CH₂)ₜ-where t is 2, 3, 4 or 5;
m, q and v are each independently 0, 1 or 2;
R₁₂ is hydrogen, C₁-C₆alkyl, C₁-C₆haloalkyl, C₁-C₆alkoxy, C₁-C₆haloalkoxy, C₁-C₆alkylthio, C₁-C₆haloalkylthio
or
NR₁₃R₁₄;
R₃ is hydrogen, C₁-C₆alkyl, C₁-C₆haloalkyl or C(O)R₁₅;
R₁₅ is C₁-C₆alkyl, C₁-C₆haloalkyl, C₁-C₆alkoxy or C₁-C₆haloalkoxy; and
R₅ is C₁-C₆alkyl,
phenyl optionally substituted with any combination of from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups, or
benzyl optionally substituted on the phenyl ring with any combination of from one to three halogen, C₁-C₆alkyl, C₁-C₆haloalkyl, C₁-C₆alkoxy, C₁-C₆haloalkoxy, C₁-C₆alkylthio or C₁C₆haloalkylthio groups; and the optical isomers thereof and the agriculturally acceptable salts thereof.

### DETAILED DESCRIPTION OF THE INVENTION

The present invention provides a method for the control of insect or acarid pests which comprises contacting said pests or their food supply, habitat or breeding grounds with a pesticidally effective amount of a 2-aryl-Δ²-1,3,4-(oxa or thia)diazoline compound of formula I.

The present invention also provides a method for the protection of growing plants from attack or infestation by insect or acarid pests which comprises applying to the foliage of the plants, or to the soil or water in which they are growing, a pesticidally effective amount of a 2-aryl-Δ²-1,3,4-(oxa or thia)diazoline compound of formula I

The pesticidal 2-aryl-Δ²-1,3,4-(oxa and thia)-diazoline compounds of the present invention have the structural formula I 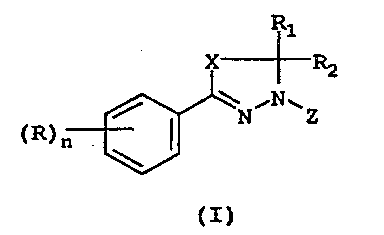 wherein n, R, R₁, R₂, X and Z are as described hereinabove for formula I.

Preferred 2-aryl-Δ²-1,3,4-oxadiazoline compounds of the present invention are those having the structural formula II 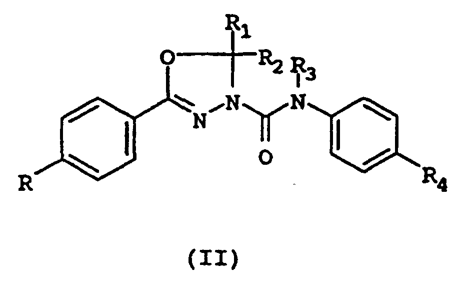 wherein
R is halogen, C₁-C₄haloalkyl, C₁-C₄haloalkoxy or phenoxy optionally substituted with any combination of from one to three halogen, C₁-C₄alkyl, C₁-C₄haloalkyl, C₁-C₄alkoxy or C₁-C₄haloalkoxy groups;
R₄ is C₁-C₄haloalkyl, C₁-C₄haloalkoxy or C₁-C₄haloalkylthio;
R₁ is C₁-C₄alkyl;
R₂ is C₁-C₄alkyl, C₁-C₄haloalkyl, (CH₂)ᵥC(O)R₁₂ or 2-pyridyl optionally substituted with any combination of from one to three halogen, C₁-C₄alkyl, C₁-C₄haloalkyl, C₁-C₄alkoxy or C₁-C₄haloalkoxy groups;
v is 0 or 1;
R₁₂ is C₁-C₄alkoxy or C₁-C₄haloalkoxy;
R₃ is hydrogen or C(O)R₁₅; and
R₁₅ is C₁-C₄alkoxy.

More preferred insecticidal and acaricidal agents of the present invention are those having the structural formula II wherein
R is F, Br, Cl or phenoxy;
R₄ is CF₃, OCF₃ or SCF₃;
R₁ is CH₃;
R₂ is CH₃, CH₂Cl, CH₂CF₃, CF₃, CH₂CO₂CH₃ or 2-pyridyl; and
R₃ is hydrogen or CO₂CH₃.

Compounds of this invention which are particularly effective insecticidal agents include
2-(*p*-chlorophenyl)-5,5-dimethyl-4'-(trifluoromethoxy)-Δ²-1,3,4-oxadiazoline-4-carboxanilide;
2-(*p*-chlorophenyl)-5,5-dimethyl-4'-(trifluoromethyl)-Δ²-1,3,4-oxadiazoline-4-carboxanilide;
2-(*p*-bromophenyl)-5,5-dimethyl-4'-(trifluoromethyl)-Δ²-1,3,4-oxadiazoline-4-carboxanilide;
2-(*p*-fluorophenyl)-5,5-dimethyl-4'-(trifluoromethyl)-Δ²-1,3,4-oxadiazoline-4-carboxanilide;
5,5-dimethyl-2-(*p*-phenoxyphenyl)-4'-[(trifluoromethyl)thio]-Δ²-1,3,4-oxadiazoline-4-carboxanilide;
2-(*p*-chlorophenyl)-5-methyl-4'-(trifluoromethoxy)-5-(trifluoromethyl)-Δ²-1,3,4-oxadiazoline-4-carboxanilide;
5-(chloromethyl)-2-(*p*-chlorophenyl)-5-methyl-4'-(trifluoromethyl)-Δ²-1,3,4-oxadiazoline-4-carboxanilide;
4,5-bis(trifluoromethyl)-2-(*p*-fluorophenyl)-5-methyl-Δ²-1,3,4-oxadiazoline-4-carboxanilide;
5-(chloromethyl)-2-(*p*-fluorophenyl)-5-methyl-4'-(trifluoromethyl)-Δ²-1,3,4-oxadiazoline-4-carboxanilide;
5-(chloromethyl)-2-(*p*-fluorophenyl)-5-methyl-4'-(trifluoromethoxy)-Δ²-1,3,4-oxadiazoline-4-carboxanilide;
2-(*p*-bromophenyl)-5-(chloromethyl)-5-methyl-4'-(trifluoromethoxy)-Δ²-1,3,4-oxadiazoline-4-carboxanilide;
2-(*p*-chlorophenyl)-5-methyl-5-(2,2,2-trifluoroethyl)-4'-(trifluoromethyl)-Δ²-1,3,4-oxadiazoline-4-carboxanilide;
2-(*p*-chlorophenyl)-5-methyl-5-(2,2,2-trifluoroethyl)-4'-(trifluoromethoxy)-Δ²-1,3,4-oxadiazoline-4-carboxanilide;
2-(p-chlorophenyl)-5-methyl-5-(2-pyridyl)-4'-(trifluoromethyl)-Δ²-1,3,4-oxadiazoline-4-carboxanilide; 2-(p-chlorophenyl)-5-methyl-5-(2-pyridyl)-4'-(trifluoromethoxy)-Δ²-1,3,4-oxadiazoline-4-carboxanilide;
methyl N-{[2-(p-chlorophenyl)-5,5-dimethyl-Δ²-1,3,4-oxadiazolin-4-yl]carbonyl}-p-(trifluoromethoxy)-carbanilate;
methyl N-{[2-(p-chlorophenyl)-5,5-dimethyl-Δ²-1,3,4-oxadiazolin-4-yl]carbonyl}-p-(trifluoromethyl)-carbanilate; and
methyl 2-(p-chlorophenyl)-5-methyl-4-{[p-(trifluoromethoxy)phenyl]carbamoyl}-Δ²-1,3,4-oxadiazoline-5-acetate, among others.

In formula I above, 5- and 6-membered heteroaromatic rings include, but are not limited to, pyridyl, pyrazolyl, imidazolyl, triazolyl, isoxazolyl, tetrazolyl, pyrazinyl, pyridazinyl, triazinyl, furanyl, thienyl, and thiazolyl rings each optionally substituted as described in formula I above.

Exemplary of halogen hereinabove are fluorine, chlorine, bromine and iodine. The terms "C₁-C₆-haloalkyl", "C₁-C₄-haloalkyl", "C₁-C₆-haloalkoxy", "C₁-C₄-haloalkoxy", "C₁-C₆-haloalkylthio" and "C₁-C₄-haloalkylthio" are defined as a C₁-C₆-alkyl group, a C₁-C₄-alkyl group, a C₁-C₆-alkoxy group, a C₁-C₄-alkoxy group, a C₁-C₆-alkylthio group and a C₁-C₄-alkylthio group substituted with one or more halogen atoms, respectively.

Novel 2-aryl-Δ²-1,3,4-oxadiazoline compounds of the present invention are those having the structural formula II.

Formula I compounds wherein X is O and Z is 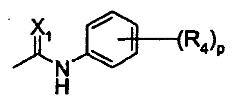 may be prepared, as
illustrated in Flow Diagram I, by reacting a hydrazine of formula III with a ketone of formula IV in the presence of a solvent such as acetone, ethanol, methylene chloride, 1,1-diethoxyethane and the like, preferably at an elevated temperature, to form a hydrazone of formula V, and reacting the formula V hydrazone with an isocyanate or isothiocyanate of formula VI in the presence of a solvent such as 1,2-dichloroethane and ethyl acetate, preferably at an elevated temperature. 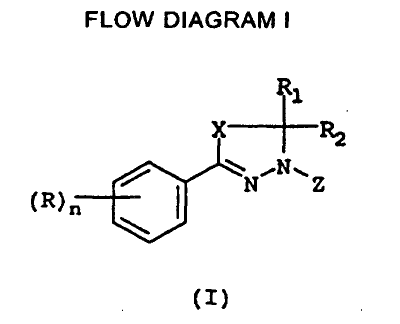 wherein n, R, R₁, R₂, X and Z are as described hereinabove, provided that: (1) R is other than CO₂R₁₀ when R is on the ortho-position of the phenyl ring, and (2) R₂ is other than ethyl or unsubstituted phenyl when X is O, n and p are 0 and R₁ is methyl.

Formula I compounds wherein X is O and
Z is 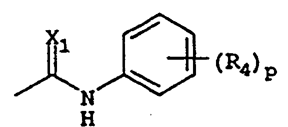 may be prepared, as
illustrated in Flow Diagram I, by reacting a hydrazine of formula III with a ketone of formula IV in the presence of a solvent such as acetone, ethanol, methylene chloride, 1,1-diethoxyethane and the like, preferably at an elevated temperature, to form a hydrazone of formula V, and reacting the formula V hydrazone with an isocyanate or isothiocyanate of formula VI in the presence of a solvent such as 1,2-dichloroethane and ethyl acetate, preferably at an elevated temperature.

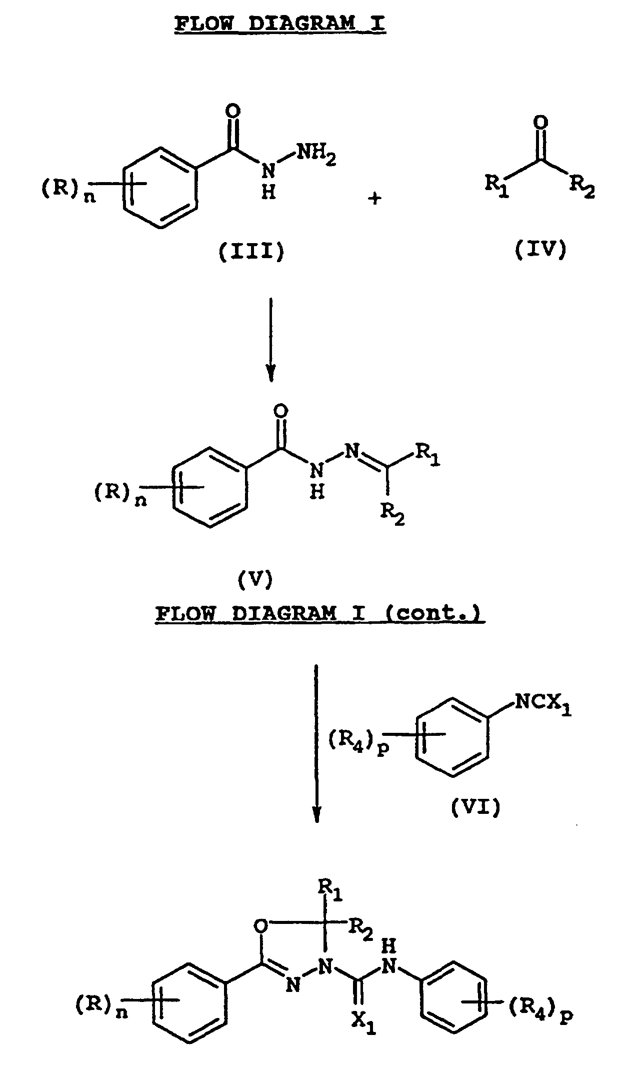

Alternatively, formula I compounds wherein X is O, R₁ is methyl, R₂ is C₁-C₆haloalkyl and
Z is 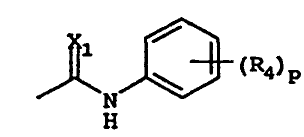 may be prepared, as shown in Flow Diagram II, by reacting a hydrazine of formula III with a 1-haloalkyl-1-acetoxyethylene compound of formula VII in the presence of a solvent such as ethanol, preferably at an elevated temperature, to obtain a hydrazone of formula VIII, and reacting the formula VIII hydrazone with an isocyanate or isothiocyanate of formula VI in the presence of a solvent such as 1,2-dichloroethane and ethyl acetate, preferably at an elevated temperature.

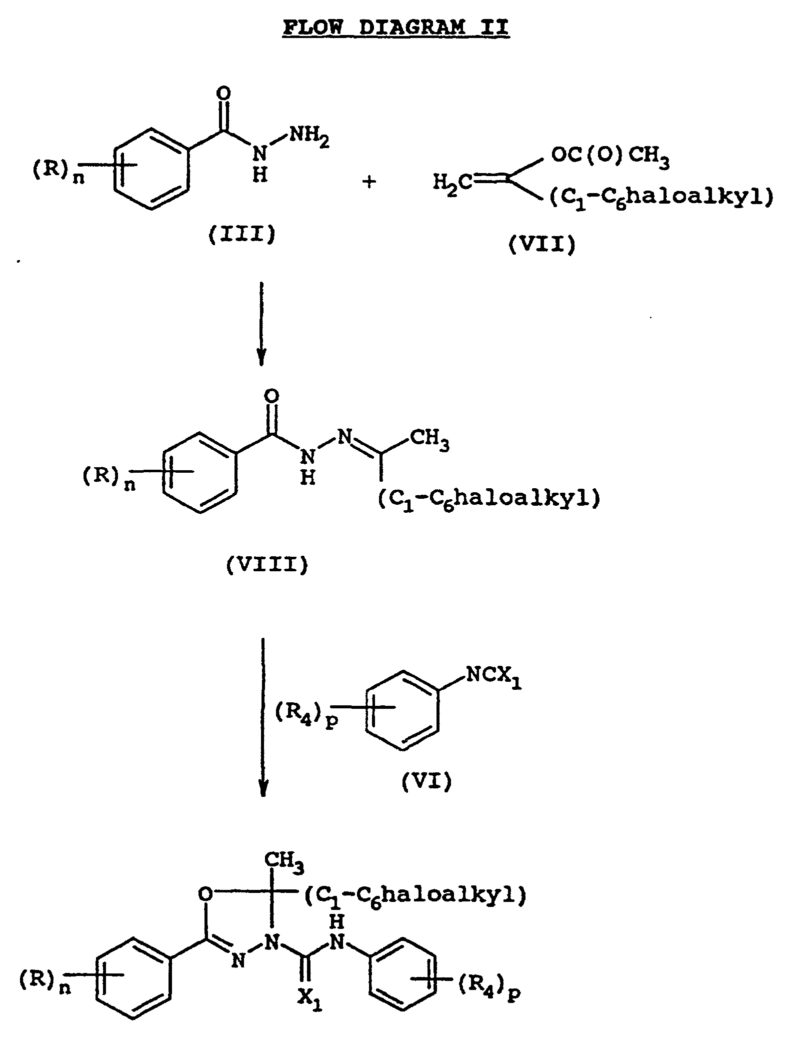

Formula I compounds wherein X is S and
Z is 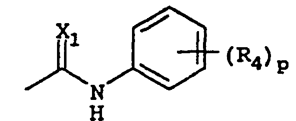 may be prepared, as
illustrated in Flow Diagram III, by reacting a hydrazine. of formula IX with a ketone of formula IV in the presence of a solvent such as acetone, ethanol, methylene chloride, 1,1-diethoxyethane and the like to form a 2-aryl-Δ²-1,3,4-thiadiazoline of formula X, and reacting the formula X compound with an isocyanate or isothiocyanate of formula VI in the presence of a solvent such as 1,2-dichlorethane and ethyl acetate.

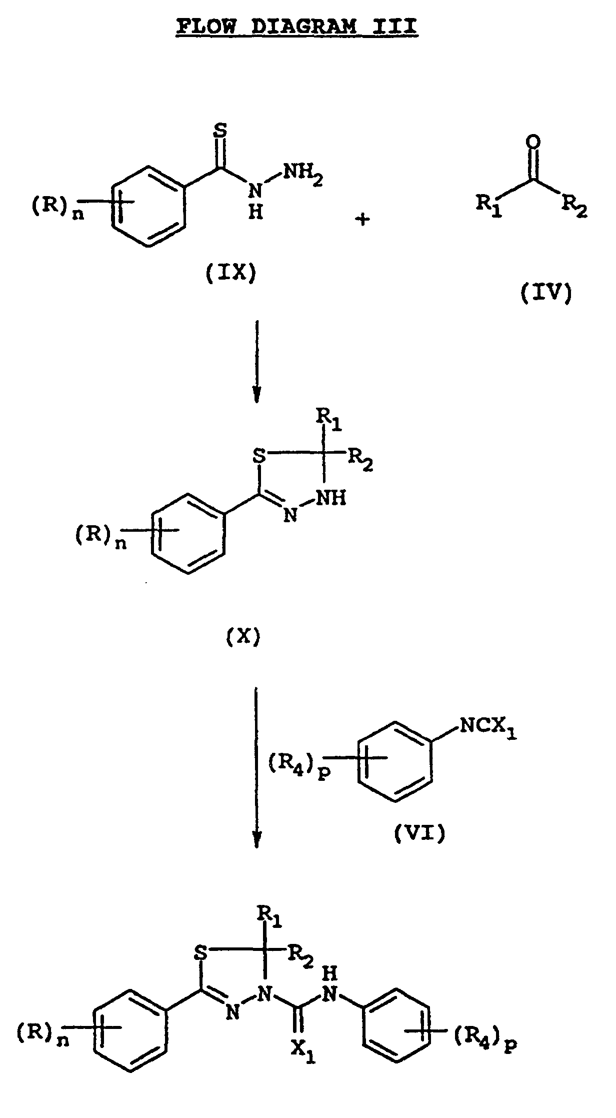

Formula I compounds wherein X is S and Z is C(X₁)R₅, C₁-C₆alkyl, C₁-C₆haloalkyl, optionally substituted benzyl or optionally substituted phenyl may be prepared, as illustrated in Flow Diagram IV, by reacting a 2-aryl-Δ²-1,3,4-thiadiazoline of formula X with a halide compound of formula XI and a base in the presence of a solvent.

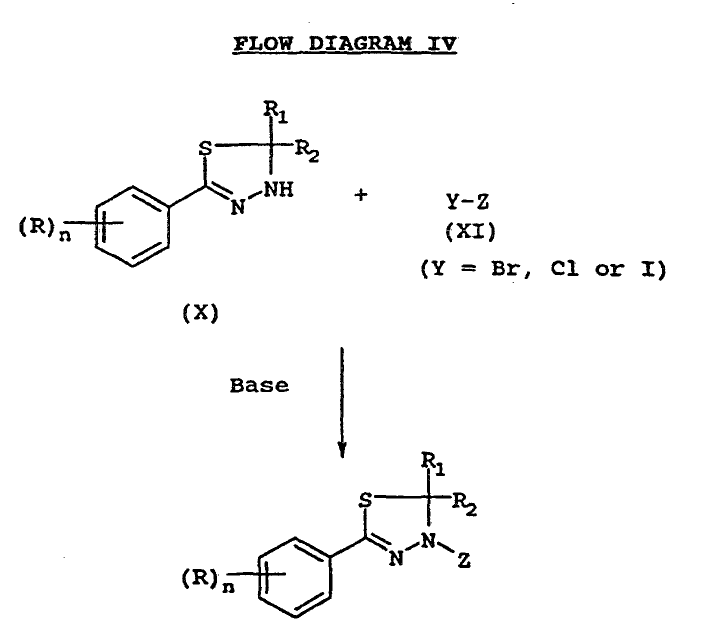

In addition, certain compounds of formula I may be converted into other compounds of formula I by using conventional procedures known to those skilled in the art.

The 2-aryl-Δ²-1,3,4-(oxa and thia)diazoline compounds of the present invention are effective for controlling insect and acarid pests. Those compounds are also effective for protecting growing or harvested crops from damage caused by insect and acarid attack and infestation.

Insects controlled by the a 2-aryl-Δ²-1,3,4-(oxa and thia)diazoline compounds of this invention include Lepidoptera such as tobacco budworms, cabbage loopers, cotton boll worms, beet armyworms, southern armyworms and diamondback moths; Homoptera such as aphids, leaf hoppers, plant hoppers and white flies; Thysanoptera such as thrips; Coleoptera such as boll weevils, Colorado potato beetles, southern corn rootworms, western corn rootworms and mustard beetles; and Orthoptera such as locusts, crickets, grasshoppers and cockroaches. Acarina controlled by the compounds of this invention include mites such as two-spotted spider mites, carmine spider mites, banks grass mites, strawberry mites, citrus rust mites and leprosis mites.

In practice generally about 10 ppm to about 10,000 ppm and preferably about 100 ppm to about 5,000 ppm of a formula I compound, dispersed in water or another liquid carrier, is effective when applied to plants or the soil in which the plants are growing to protect the plants from insect and acarid attack and infestation.

The 2-aryl-Δ²-1,3,4-(oxa and thia)diazoline compounds of this invention are also effective for controlling insect and acarid pests when applied to the foliage of plants and/or to the soil or water in which said plants are growing in sufficient amount to provide a rate of about 0.1 kg/ha to 4.0 kg/ha of active ingredient.

While the compounds of this invention are effective for controlling insect and acarid pests when employed alone, they may also be used in combination with other biological agents, including other insecticides and acaricides. For example, the formula I compounds of this invention may be used effectively in conjunction or combination with pyrethroids, phosphates, carbamates, cyclodienes, endotoxin of *Bacillus thuringiensis* (Bt), formamidines, phenol tin compounds, chlorinated hydrocarbons, benzoylphenylureas, pyrroles and the like.

The compounds of this invention may be formulated as emulsifiable concentrates, flowable concentrates or wettable powders which are diluted with water or other suitable polar solvent, generally *in situ,* and then applied as a dilute spray. Said compounds may also be formulated in dry compacted granules, granular formulations, dusts, dust concentrates, suspension concentrates, microemulsions and the like all of which lend themselves to seed, soil, water and/or foliage applications to provide the requisite plant protection. Such formulations or compositions of the present invention include a compound of the invention (or combinations thereof) admixed with one or more agronomically acceptable inert, solid or liquid carriers. Those compositions contain a pesticidally effective amount of said compound or compounds, which amount may vary depending upon the particular compound, target pest, and method of use. Those skilled in the art can readily determine what is a pesticidally effective amount without undue experimentation.

In order to facilitate a further understanding of the invention, the following examples are presented primarily for the purpose of illustrating more specific details thereof. The scope of the invention should not be deemed limited by the examples, but encompasses the entire subject matter defined in the claims.

### EXAMPLE 1

### Preparation of 2-(α,α,α-Trifluoro-m-tolyl)-5,5-dimethyl-4'-(trifluoromethoxy)-Δ²-1,3,4-oxadiazoline-4-carboxanilide

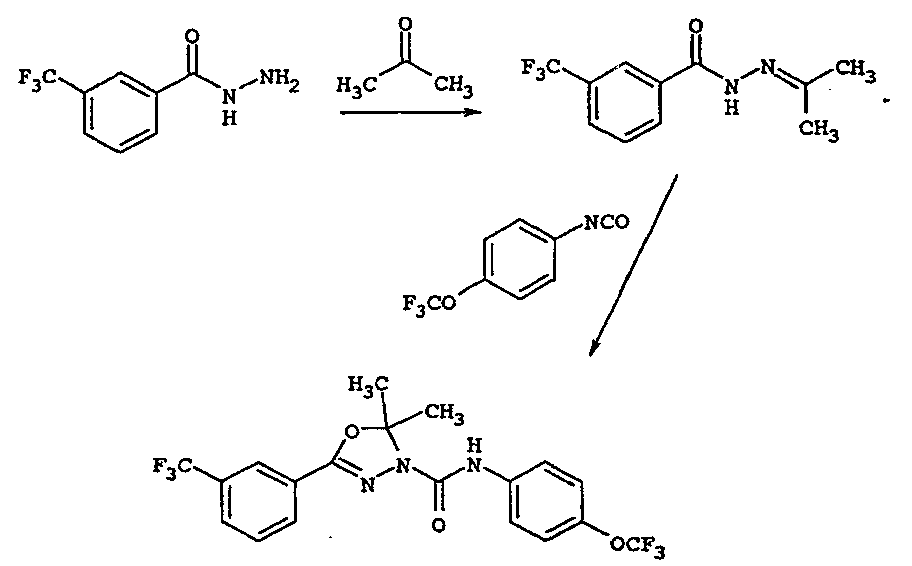

A solution of *m*-trifluoromethylbenzoyl hydrazine (1.84 g) and acetone (40 mL) is refluxed for 48 hours, cooled to room temperature and concentrated in vacuo to obtain a colorless hydrazone (1.48 g, m.p. 100-103°C). A solution of the hydrazone (0.74 g), *p*-trifluoromethoxyphenylisocyanate (0.62 g), and 1,2-dichloroethane (15 mL) is refluxed for 16 hours, cooled to room temperature, and concentrated *in vacuo* to give the title product as a colorless solid (1.28 g, m.p. 120-122°C).

Using essentially the same procedure as described for the preparation of Example 1, but using the appropriately substituted hydrazine, ketone and isocyanate, the following compounds are obtained:

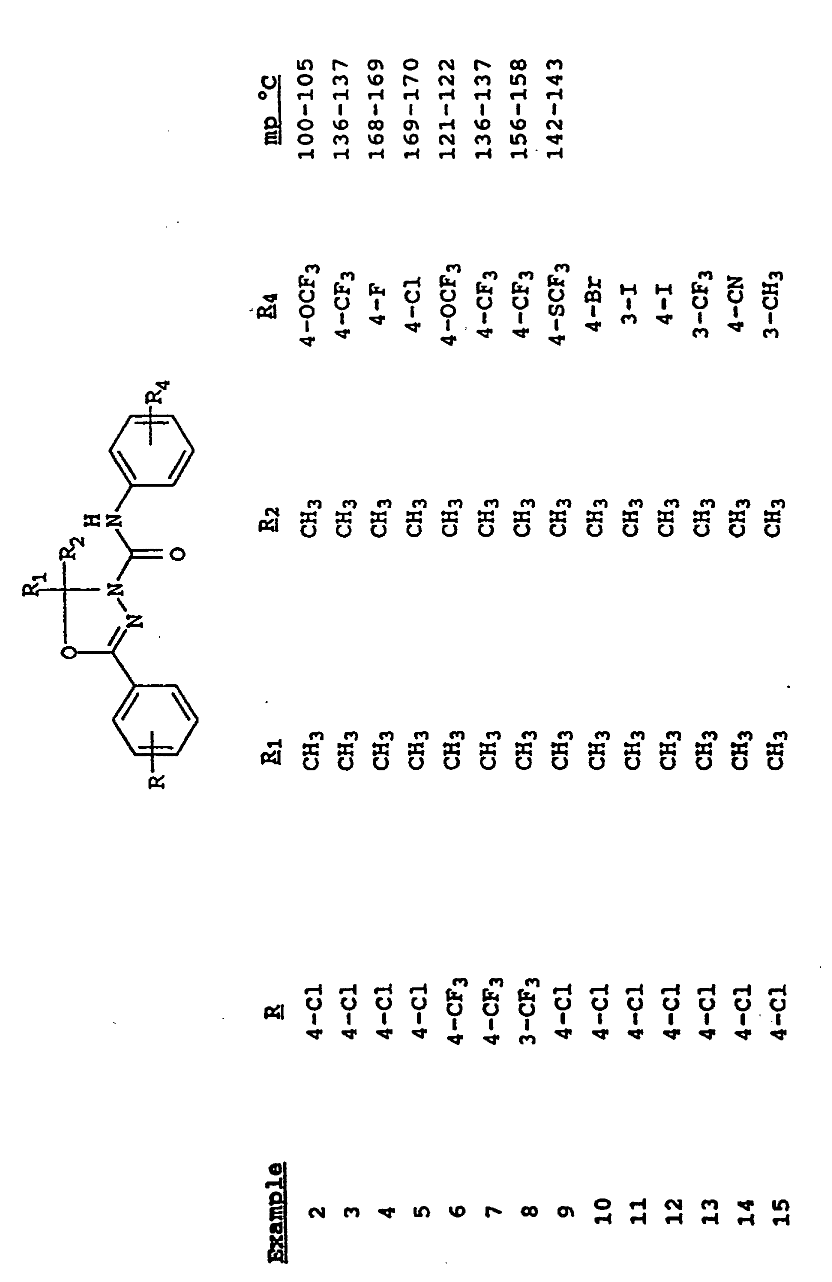

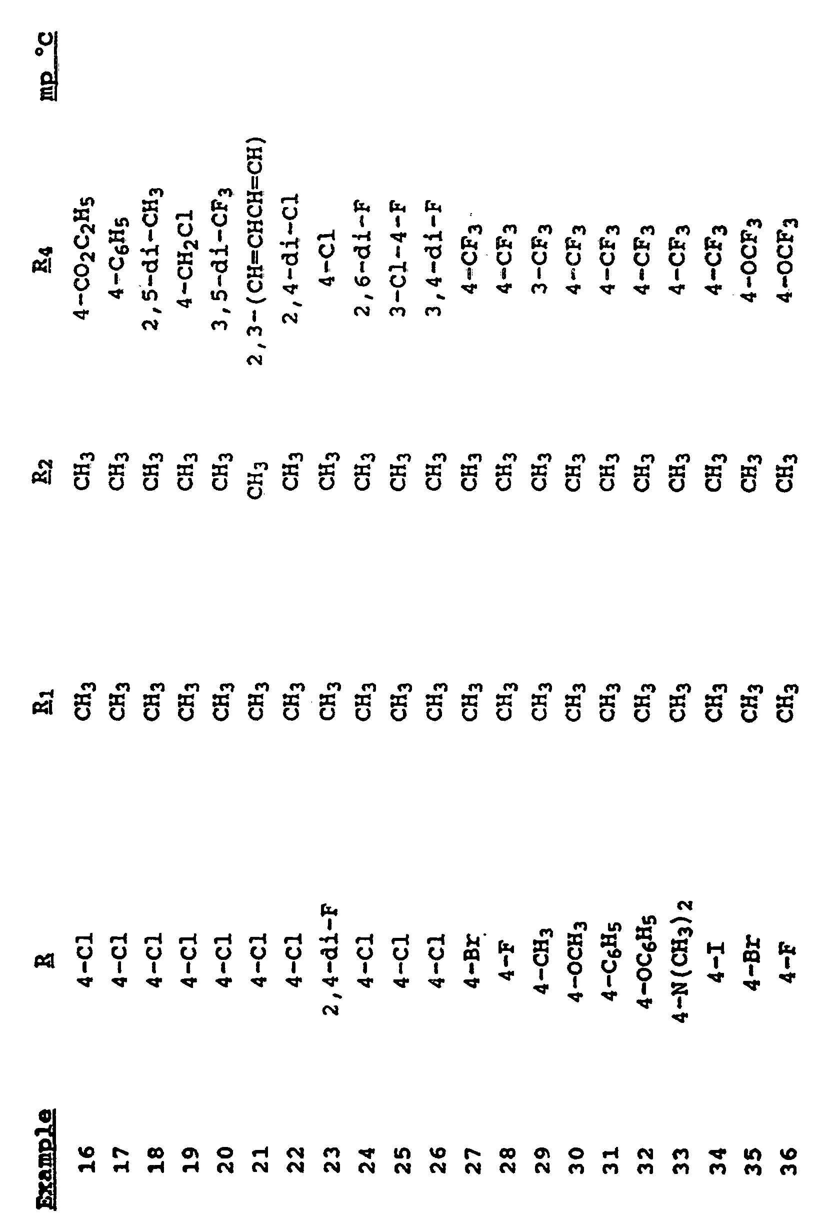

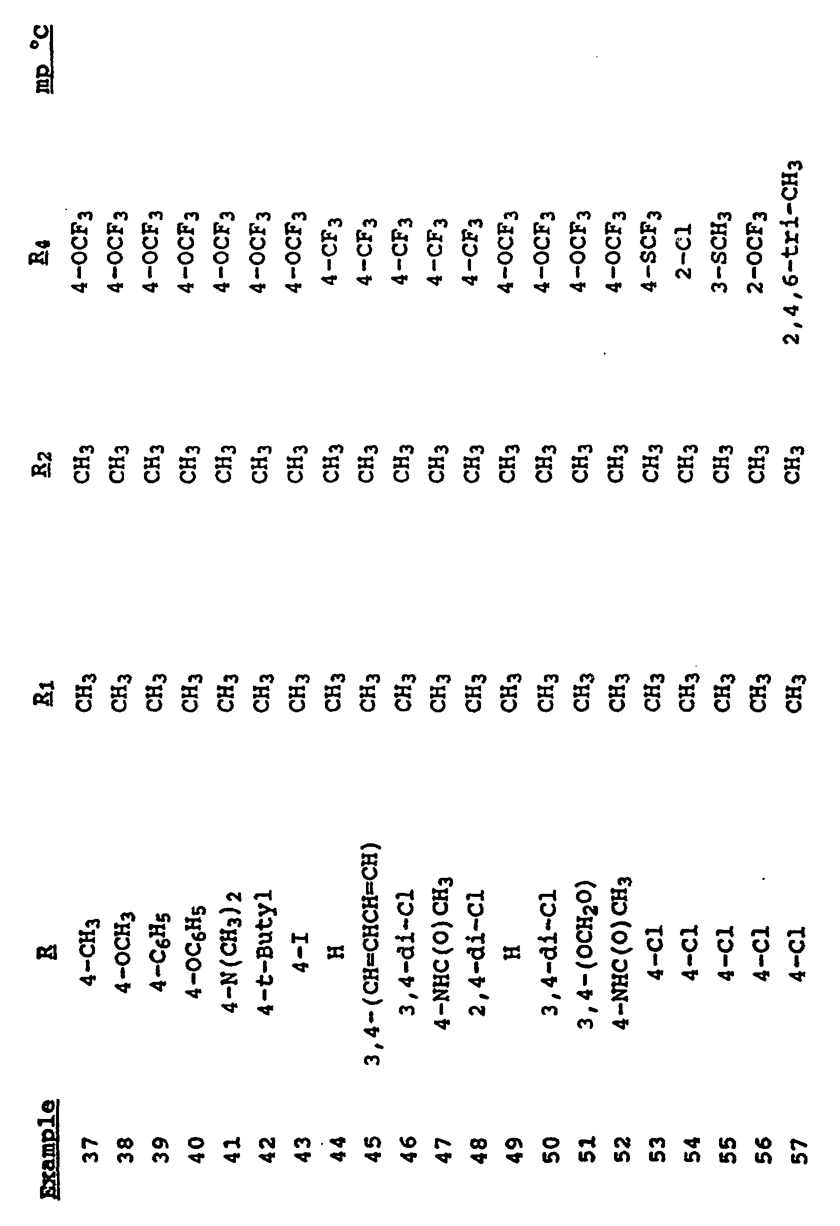

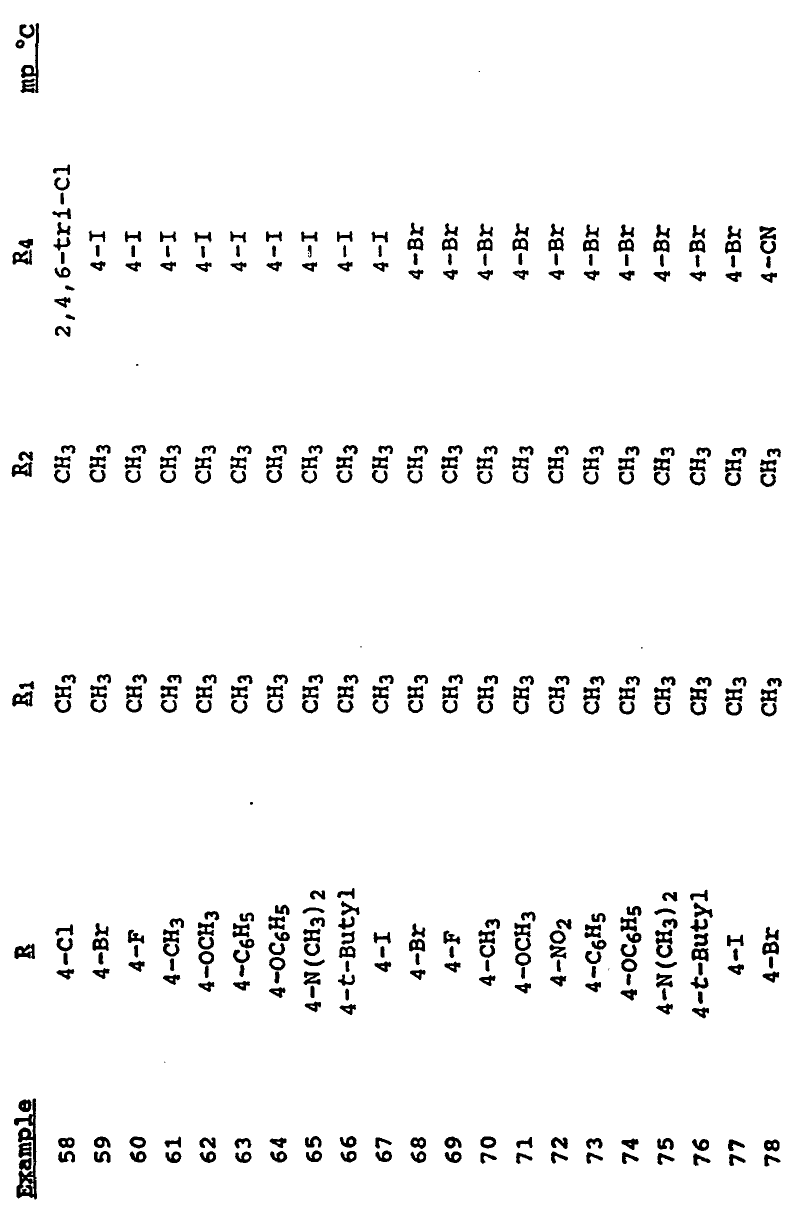

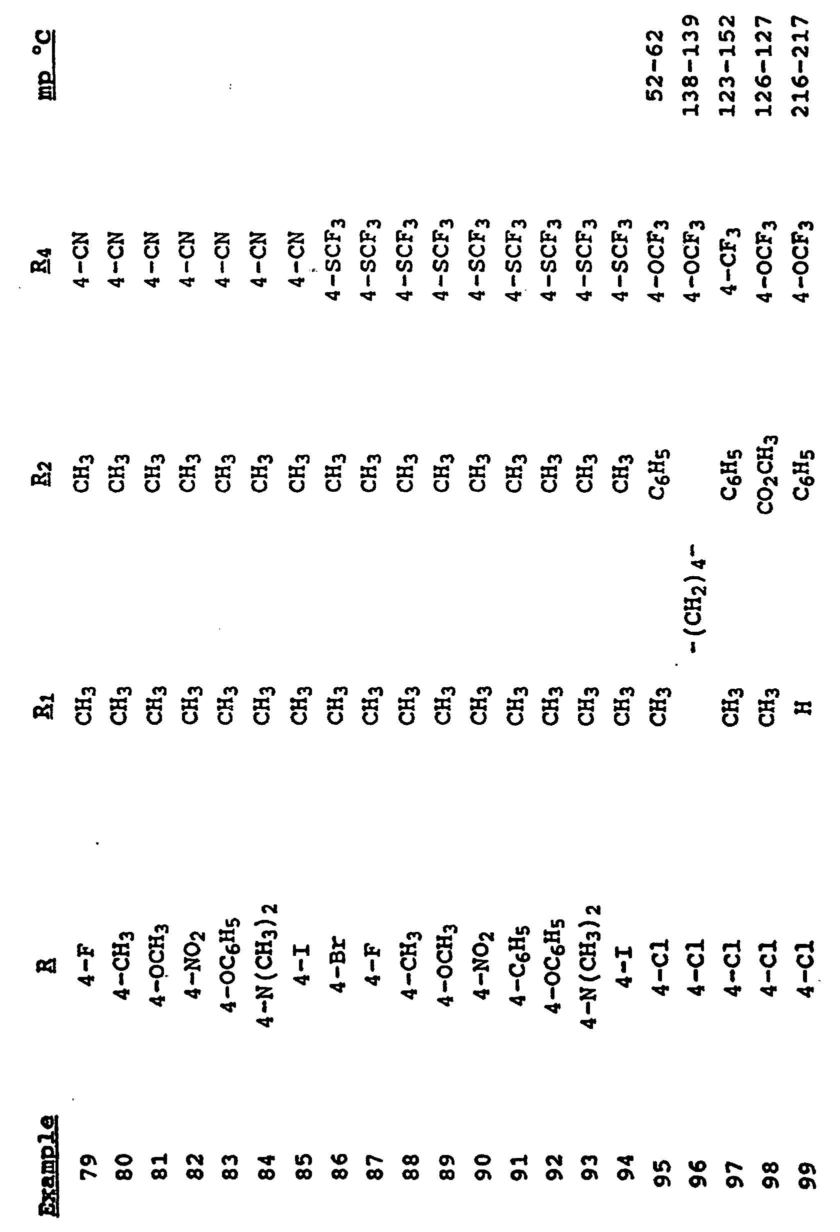

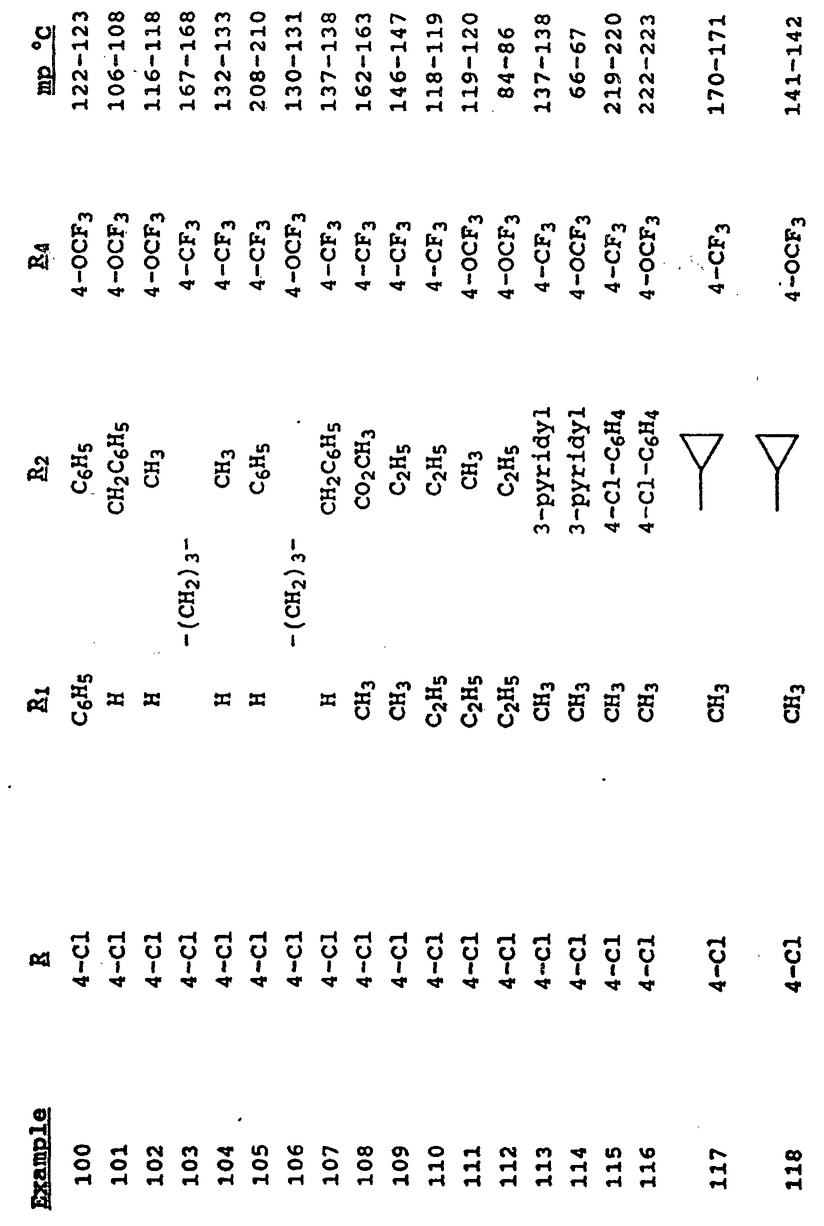

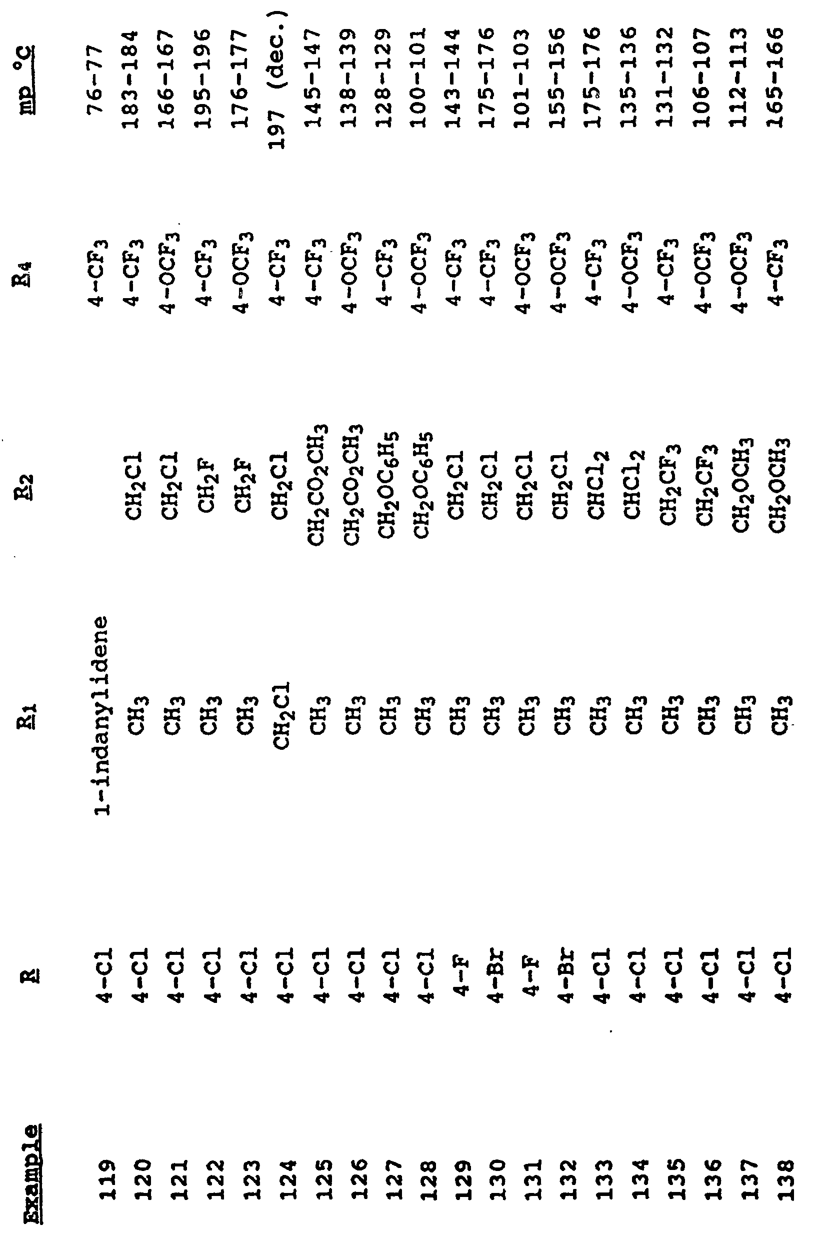

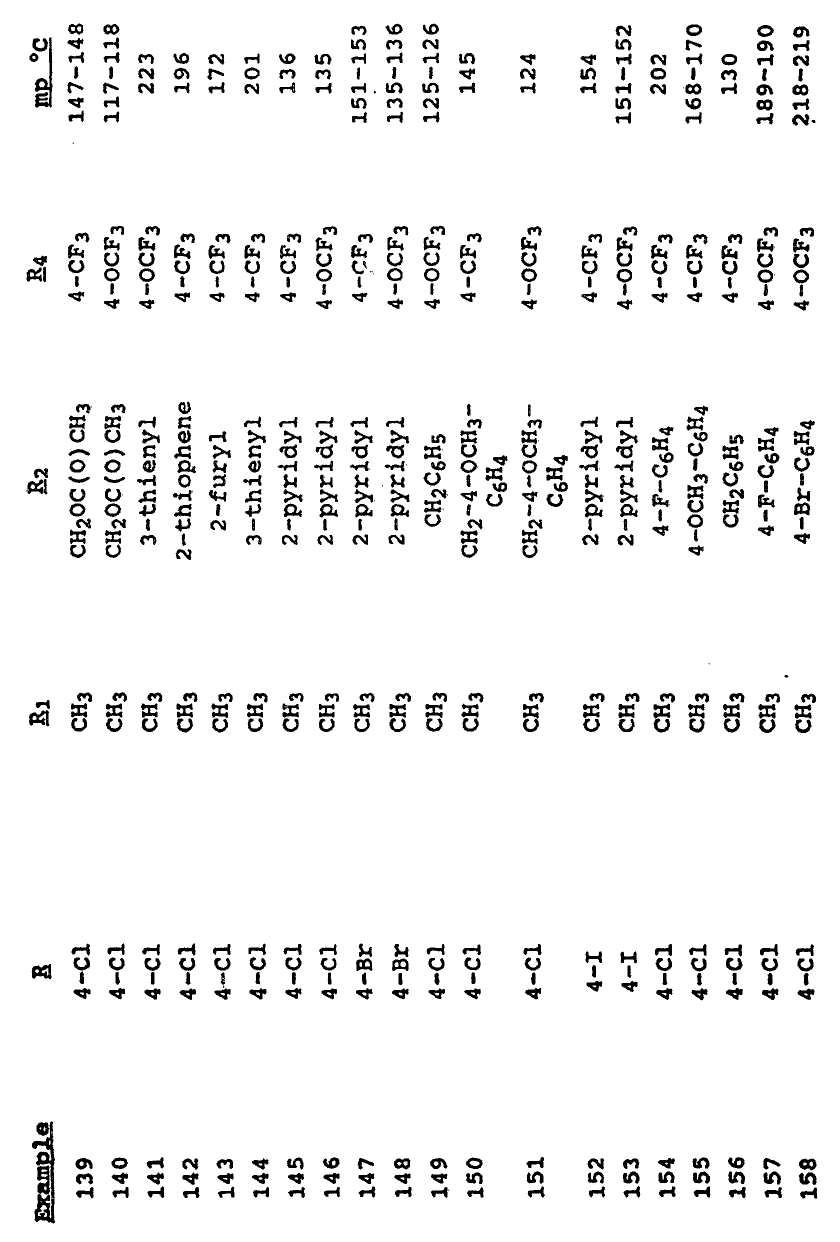

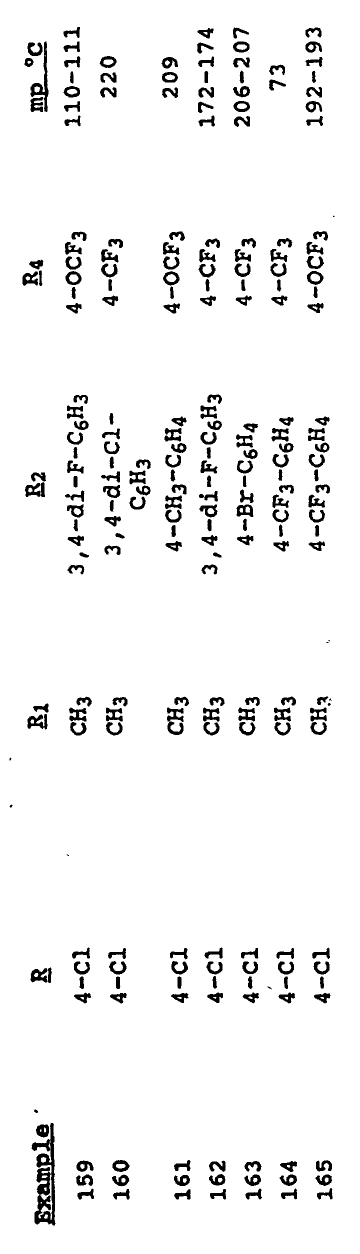

### EXAMPLE 166

### Preparation of 2-( p-Chlorophenyl)-5-methyl-5-trifluoromethyl-4'-(trifluoromethyl)-Δ²-1,3,4-oxadiazoline-4-carboxanilide

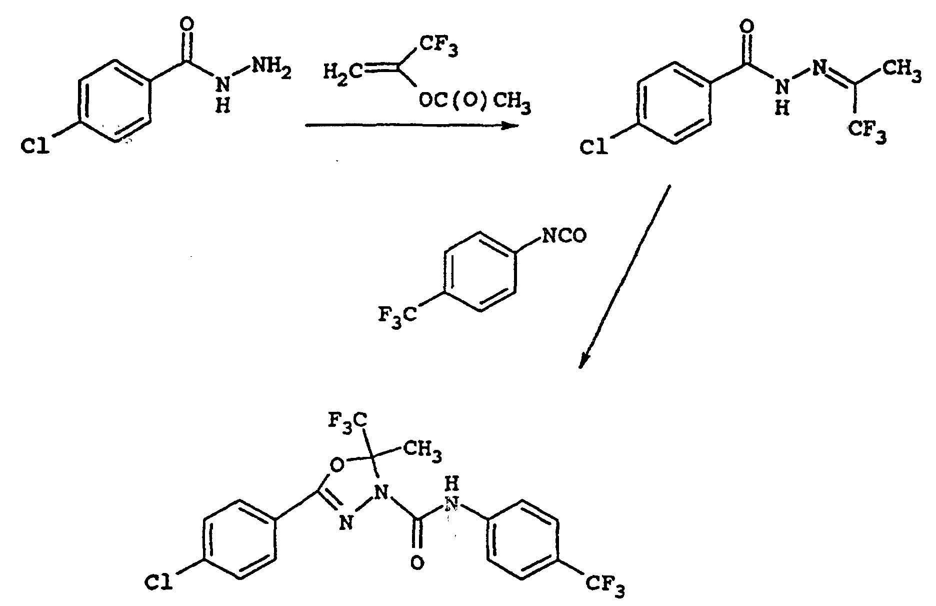

A mixture of *p*-chlorobenzoyl hydrazine (1.77 g), 1-trifluoromethyl-1-acetoxyethylene (1.78 g) and ethanol (35 mL) is refluxed for 17 hours, cooled to room temperature, and concentrated *in vacuo* to obtain the corresponding benzoyl hydrazone (0.71 g). A mixture of the hydrazone (0.8 g) and 1,2-dichloroethane (10 mL) is treated with a *p*-trifluoromethylphenylisocyanate (0.67 g), heated at reflux for 87 hours, and concentrated *in vacuo* to obtain a colorless solid (1.48 g). Flash chromatography of the solid on silica gel (25% CH₂Cl₂/hexanes to 50% CH₂Cl₂/hexanes) gives the title product as a colorless solid (0.16 g, m.p. 157-158°C).

Using essentially the same procedure as described for Example 166, but using the appropriately substituted hydrazine and isocyanate, the following compounds are obtained.

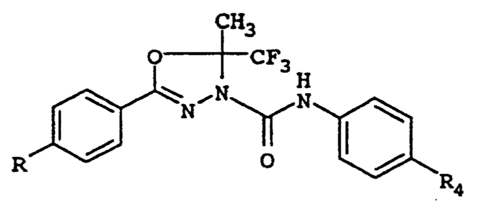

| **Example** | **R** | **R**_{**4**} | **mp °C** |
|---|---|---|---|
| 167 | Cl | OCF₃ | 128-129 |
| 168 | Br | CF₃ | 156-157 |
| 169 | F | CF₃ | 141-142 |

### EXAMPLE 170

### Preparation of p-chlorobenzoylthiohydrazide

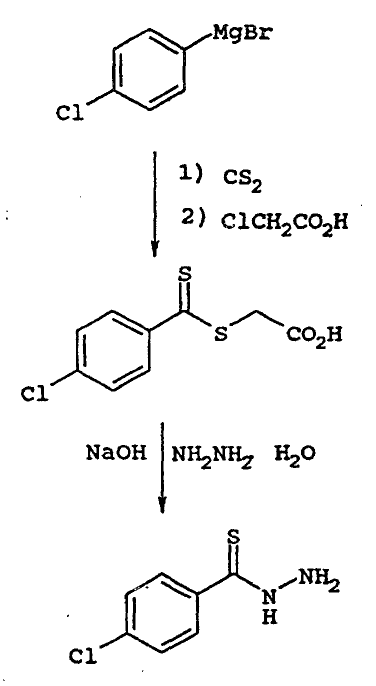

A solution of carbon disulfide (4.5 mL, 75 mmol) and tetrahydrofuran (50 mL) is cooled to 0°C, treated dropwise with a solution *p*-chlorophenylmagnesium bromide (50 mL of 1M solution) at a rate that maintains the temperature below 10°C, warmed to and stirred at room temperature for 2 hours, concentrated *in vacuo* and diluted with water. The resultant aqueous mixture is filtered through diatomaceous earth. The filtrate is treated with a solution of chloroacetic acid (5.67 g), sodium hydrogen carbonate (3.82 g) and water (24 mL), stirred for three days at room temperature, acidified to pH 1 with 50% aqueous sulfuric acid and filtered to obtain the thioester (8.98 g). To a cold (0°C) solution of the thioester (3.5 g), sodium hydroxide (0.58 g) and water (35 mL) is added hydrazine hydrate (1.4 g). During the addition, the color changes from red to yellow and a solid precipitates. The solid is collected, washed with water, and dried to give the title product (1.92 g, m.p. 112-114°C).

### EXAMPLE 171

### Preparation of 2-(p-chlorophenyl)-5,5-dimethyl-Δ²-1,3,4-thiadiazoline

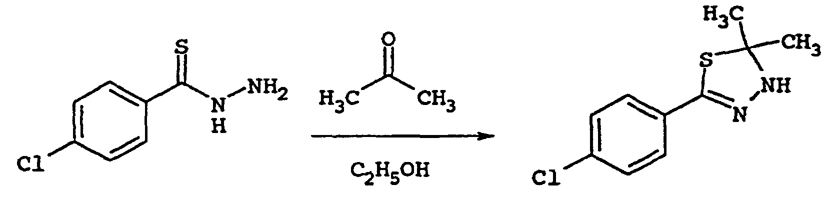

A solution of *p*-chlorobenzoylthiohydrazine (1.02 g), acetone (1.89 g) and ethanol (5 mL) is stirred at room temperature for 4 days and the solvents are evaporated to obtain a brown solid. Flash chromatography of the brown solid on silica gel (10% ethyl acetate/hexanes) gives the title product as a yellow solid (0.44 g, m.p. 51-53°C).

### EXAMPLE 172

### Preparation of 2-(p-Chlorophenyl)-5,5-dimethyl-4'-(trifluoromethyl)-Δ²-1,3,4-thiadiazoline-4-carboxanilide

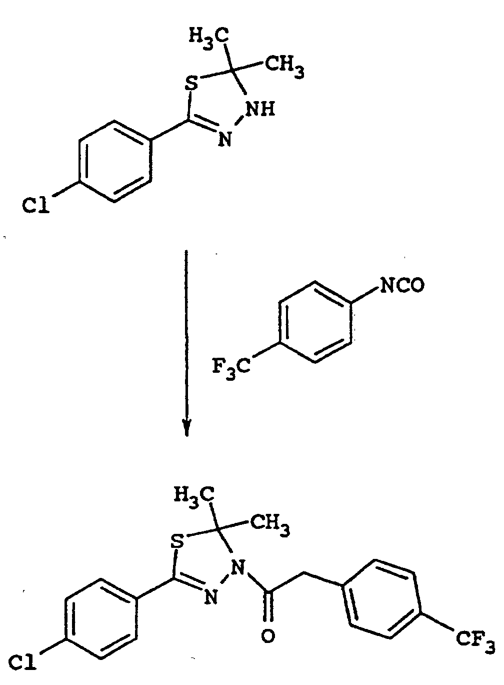

A solution of 2-(*p*-chlorophenyl)-5,5-dimethyl-Δ²-1,3,4-thiadiazoline (0.33 g) and 1,2-dichloroethane (8 mL) is treated with *p*-trifluoromethylphenylisocyanate (0.30 g), stirred for 72 hours at room temperature, and concentrated *in vacuo* to obtain a solid. Flash chromatography of the solid on silica gel (30% methylene chloride/hexanes) gives the title product as a colorless solid (0.61 g, m.p. 129-131°C).

Using essentially the same procedure as described for Example 172, but using the appropriately substituted isocyanate, the following compound is obtained: 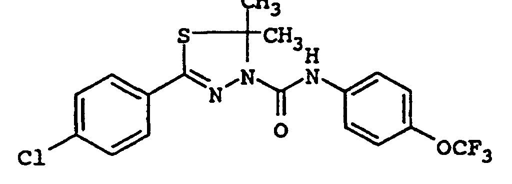 Example 173 mp 102-103°C

### EXAMPLE 174

### Preparation of 1-Oxide-2-(p-chlorophenyl)-5,5-dimethyl-4'-(trifluoromethoxy)-Δ²-1,3,4-thiadiazoline-4-carboxanilide

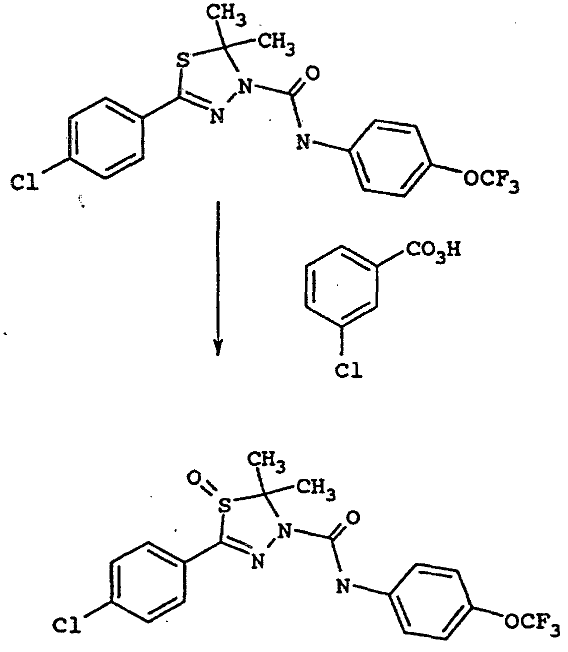

A solution of 2-(*p*-chlorophenyl)-5,5-dimethyl-4'-(trifluoromethoxy)-Δ²-1,3,4-thiadiazoline-4-carboxanilide (0.50 g) and dichloromethane (15 mL) is stirred at -5° C, treated with 3-chloroperoxybenzoic acid (0.30 g, 70%), stirred for 3.5 hours at room temperature, and diluted with dichloromethane (10 mL). The resultant mixture is washed with 5% sodium carbonate solution, dried over anhydrous magnesium sulfate, concentrated to 10 mL volume, and cooled in a refrigerator overnight. The white precipitate is filtered and dried to give the title product as a colorless solid (0.49 g, m.p. 214-215°C).

### EXAMPLE 175

### Preparation of 1,1-Dioxide-2-(p-chlorophenyl)-5,5-dimethyl-4'-(trifluoromethoxy)-Δ²-1,3,4-thiadiazoline-4-carboxanilide

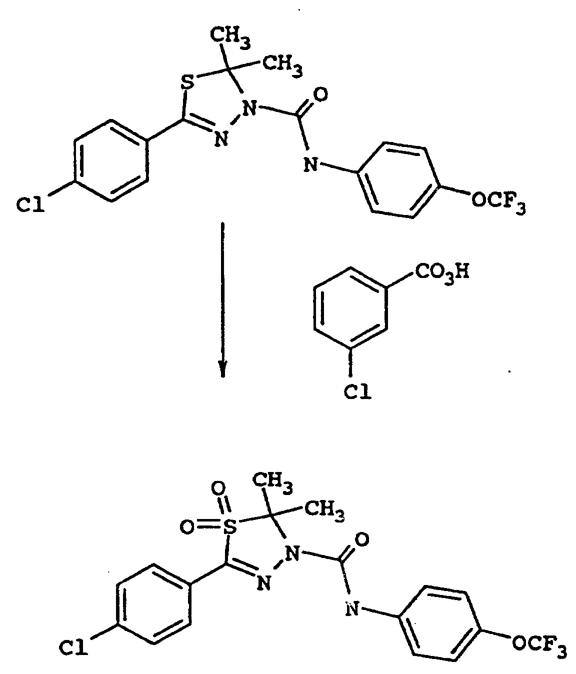

A solution of 2-(*p*-chlorophenyl)-5,5-dimethyl-4'-(trifluoromethoxy)-Δ²-1,3,4-thiadiazoline-4-carboxanilide (0.50 g) and dichloromethane (15 mL) is stirred at -5° C, treated with 3-chloroperoxybenzoic acid (1.79 g, 70%), stirred for 18 hours at room temperature, treated with additional 3-chloroperoxybenzoic acid (0.12 g, 70%), stirred for 14 hours at room temperature, washed with 5% sodium carbonate solution, dried over anhydrous magnesium sulfate, and concentrated *in vacuo* to obtain a solid. Flash chromatography of the solid on silica gel using a 10% ethyl acetate in hexanes solution gives the title product as a colorless solid (0.42 g, m.p. 181°C).

### EXAMPLE 176

### Insecticidal and acaricidal evaluation of test compounds

Test solutions are prepared by dissolving the test compound in a 35% acetone in water mixture to give a concentration of 10,000 ppm. Subsequent dilutions are made with water as needed.

### Spodoptera eridania, 2nd instar larvae, southern armyworm (SAW)

A Sieva lima bean leaf expanded to 7-8 cm in length is dipped in the test solution with agitation for 3 seconds and allowed to dry in a hood. The leaf is then placed in a 100 x 10 mm petri dish containing a damp filter paper on the bottom and ten 2nd instar caterpillars. At 5 days, observations are made of mortality, reduced feeding, or any interference with normal molting.

### Diabrotica virgifera virgifera Leconte, 2nd instar western corn rootworm (WCR)

One cc of fine talc is placed in a 30 mL wide-mouth screw-top glass jar. One mL of the appropriate acetone test solution is pipetted onto the talc so as to provide 1.25 mg of active ingredient per jar. The jars are set under a gentle air flow until the acetone is evaporated. The dried talc is loosened, 1 cc of millet seed is added to serve as food for the insects and 25 mL of moist soil is added to each jar. The jar is capped and the contents thoroughly mixed mechanically. Following this, ten 2nd instar rootworms are added to each jar and the jars are loosely capped to allow air exchange for the larvae. The treatments are held for 5 days when mortality counts are made. Missing larvae are presumed dead, since they decompose rapidly and cannot be found. The concentrations of active ingredient used in this test correspond approximately to 50 kg/ha.

### Tetranychus urticae (OP-resistant strain), 2-spotted spider mite (TSM)

Sieva lima bean plants with primary leaves expanded to 7-8 cm are selected and cut back to one plant per pot. A small piece is cut from an infested leaf taken from the main colony and placed on each leaf of the test plants. This is done about 2 hours before treatment to allow the mites to move over to the test plant to lay eggs. The size of the cut, infested leaf is varied to obtain about 100 mites per leaf. At the time of test treatment, the piece of leaf used to transfer the mites is removed and discarded. The newly-infested plants are dipped in the test solution for 3 seconds with agitation and set in the hood to dry. After 2 days, one leaf is removed and mortality counts are made.

### Aphis gossypii, cotton aphid (CA)

Cotton plants at the cotyledon stage are selected and cut back to one plant per pot. A heavily infested leaf is taken from the main colony and placed on top of each cotyledon. The aphids are allowed to transfer to the host plant overnight. At the time of test treatment, the leaf used to transfer the aphids is removed and discarded. The cotyledons are dipped in the test solution and allowed to dry. After 5 days, mortality counts are made.

### Diabrotica undecimpunctata howardi, eggs-southern corn rootworm (SCR-Eggs)

Wells containing artificial diet are treated with the test solutions and dried. Southern corn rootworm eggs are then placed in the wells. The wells are covered with vented, adhesive, clear plastic covers. After 7 days, mortality counts are made.

### Heliothis virenscens, 3rd instar tobacco budworm (TBW)

Cotton cotyledons are dipped in the test solution and allowed to dry in a hood. When dry, each is cut into quarters and ten sections are placed individually in 30 mL plastic medicine cups containing a 5 to 7 mm long piece of damp dental wick. One 3rd instar caterpillar is added to each cup and a cardboard lid placed on the cup. Treatments are maintained for 3 days before mortality counts and estimates of reduction in feeding damage are made.

The tests are rated according to the scale shown below and the data obtained are shown in Table I.

| **Rating scale** | |
|---|---|
| 0 = no effect | 5 = 56-65% kill |
| 1 = 10-25% kill | 6 = 66-75% kill |
| 2 = 26-35% kill | 7 = 76-85% kill |
| 3 = 36-45% kill | 8 = 86-99% kill |
| 4 = 46-55% kill | 9 = 100% kill |

**TABLE I**

| **Insecticidal and Acaricidal Evaluations** | | | | | | |
|---|---|---|---|---|---|---|
| **Ex.** | **CA (300**^{**1**}**)** | **SAW (300**^{**1**}**)** | **TBW (300**^{**1**}**)** | **TSM (300**^{**1**}**)** | **SCR Eggs (1000**^{**1**}**)** | **WCR (50**^{**1**}**)** |
| 1 | 0 | 9 | 4 | 0 | 9 | 0 |
| 2 | | 9 | 9 | 0 | 9 | |
| 3 | 0 | 9 | 9 | 0 | 9 | 4 |
| 4 | 0 | 4 | | 0 | 0 | 0 |
| 5 | 0 | 9 | 3 | 2 | 9 | 0 |
| 6 | 0 | 9 | 9 | 0 | 9 | 1 |
| 7 | 0 | 9 | 8 | 0 | 9 | 0 |
| 8 | 0 | 0 | | 0 | 0 | 0 |
| 9 | 0 | 9 | 9 | 0 | 9 | 2 |
| 10 | 0 | 9 | 3 | 0 | 9 | 0 |
| 11 | 0 | 0 | | 9 | 0 | 0 |
| 12 | 0 | 9 | 9 | 0 | 9 | 0 |
| 13 | 0 | 7 | 0 | 0 | 0 | 0 |
| 14 | 0 | 9 | 9 | 0 | 9 | 0 |
| 15 | 0 | 0 | | 4 | 0 | 0 |
| 16 | 0 | 8 | 0 | 0 | 9 | 0 |
| 17 | 0 | 9 | 0 | 0 | 0 | 0 |
| 18 | 0 | 0 | | 0 | 0 | 1 |
| 19 | | | | | 0 | |
| 20 | | | | | 0 | |
| 21 | | | | | 0 | |
| 22 | 0 | 1 | | 0 | 0 | 0 |
| 23 | 0 | 8 | 0 | 0 | 0 | 1 |
| 24 | 0 | 2 | | 0 | 0 | 0 |
| 25 | 0 | 2 | | 3 | 0 | 0 |
| 26 | 0 | 0 | | 0 | 0 | 1 |
| 27 | 0 | 9 | 9 | 0 | 9 | 0 |
| 28 | 0 | 9 | 9 | 0 | 9 | 4 |
| 29 | 0 | 9 | 0 | 0 | 9 | 0 |
| 30 | 0 | 9 | 1 | 0 | 9 | 0 |
| 31 | 0 | 9 | 0 | 0 | 9 | 0 |
| 32 | 0 | 9 | 8 | 0 | 9 | 0 |
| 33 | 0 | 9 | 1 | 0 | 9 | 0 |
| 34 | 0 | 9 | 9 | 0 | 9 | 0 |
| 35 | 0 | 9 | 9 | 0 | 9 | 0 |
| 36 | 5 | 9 | 8 | 0 | 9 | 9 |
| 37 | 0 | 9 | 0 | 0 | 9 | 0 |
| 38 | 0 | 9 | 1 | 0 | 8 | 0 |
| 39 | 0 | 9 | 1 | 0 | 9 | 1 |
| 40 | 0 | 9 | 3 | 0 | 9 | 0 |
| 41 | 0 | 9 | 3 | 0 | 9 | 0 |
| 42 | 0 | 1 | | 0 | 9 | 0 |
| 43 | 0 | 9 | 9 | 0 | 9 | 0 |
| 44 | 0 | 9 | 0 | 0 | 9 | 0 |
| 45 | 0 | 8 | 0 | 0 | 0 | 0 |
| 46 | 0 | 8 | 0 | 0 | 0 | 0 |
| 47 | 0 | 9 | 5 | 0 | 0 | 0 |
| 48 | 0 | 9 | 6 | 0 | 9 | 0 |
| 49 | 0 | 9 | 1 | 0 | 9 | 7 |
| 50 | 0 | 9 | 0 | 0 | 0 | 0 |
| 51 | 0 | 9 | 5 | 0 | 0 | 0 |
| 52 | 0 | 9 | 0 | 0 | 0 | 0 |
| 53 | 0 | 9 | 0 | 0 | 9 | 4 |
| 54 | 0 | | | 0 | 0 | 4 |
| 55 | 0 | | | 0 | 8 | 0 |
| 56 | 0 | | | 0 | 8 | 0 |
| 57 | 8 | 0 | | 0 | 7 | 0 |
| 58 | 0 | 8 | 0 | 0 | 0 | 0 |
| 59 | 0 | 9 | 9 | 0 | 9 | 0 |
| 60 | 0 | 9 | 9 | 0 | 9 | 0 |
| 61 | 0 | 9 | 0 | 0 | 9 | 0 |
| 62 | 0 | 9 | 1 | 0 | 0 | 0 |
| 63 | 0 | 9 | 0 | 0 | 9 | 0 |
| 64 | 0 | 9 | 9 | 0 | 9 | 0 |
| 65 | 0 | 9 | 7 | 0 | 9 | 0 |
| 66 | 0 | 4 | | 0 | 0 | 0 |
| 67 | 0 | 9 | 9 | 0 | 9 | 0 |
| 68 | 0 | 8 | 1 | 0 | 8 | 9 |
| 69 | 0 | 9 | 9 | 0 | 9 | 2 |
| 70 | 0 | 3 | | 0 | 0 | 1 |
| 71 | 0 | 1 | | 0 | 0 | 3 |
| 72 | 0 | 1 | | 0 | 0 | 2 |
| 73 | | 6 | | 0 | 9 | 2 |
| 74 | 0 | 9 | 6 | 0 | 7 | 1 |
| 75 | 0 | 1 | | 0 | 7 | 9 |
| 76 | 0 | 0 | | 0 | 0 | 4 |
| 77 | 0 | 9 | 0 | 0 | 8 | 0 |
| 78 | 0 | 9 | 0 | 0 | 8 | 0 |
| 79 | 0 | 9 | 0 | 0 | 9 | 1 |
| 80 | 0 | 3 | | 0 | 0 | 9 |
| 81 | 0 | 1 | | 0 | 0 | 0 |
| 82 | 0 | 6 | | 0 | 0 | 2 |
| 83 | 0 | 3 | | 0 | 0 | 0 |
| 84 | 0 | 0 | | 0 | 0 | 0 |
| 85 | 0 | 0 | | 0 | 0 | 3 |
| 86 | 0 | 9 | 9 | 0 | | 4 |
| 87 | 0 | 9 | 8 | 0 | | 9 |
| 88 | 0 | 9 | 0 | 0 | | 0 |
| 89 | 0 | 9 | 1 | 0 | | 0 |
| 90 | 0 | 8 | 0 | 0 | | 0 |
| 91 | 0 | 9 | 7 | 0 | | 0 |
| 92 | 0 | 9 | 0 | 0 | | 0 |
| 93 | 0 | 9 | 0 | 0 | | 0 |
| 94 | 0 | 9 | 7 | 0 | | 0 |
| 95 | 0 | 9 | 9 | 0 | 9 | 0 |
| 96 | 0 | 0 | | 0 | 0 | 0 |
| 97 | 0 | 9 | 3 | 0 | 9 | 0 |
| 98 | 0 | 9 | 3 | 0 | 9 | 1 |
| 99 | 0 | 9 | 0 | 0 | 0 | 0 |
| 100 | 0 | 0 | | 0 | 0 | 0 |
| 101 | 0 | 9 | 8 | 0 | 9 | 0 |
| 102 | 0 | 9 | 6 | 0 | 7 | 0 |
| 103 | 0 | 9 | 9 | 0 | 8 | 0 |
| 104 | 0 | 9 | 2 | 0 | 8 | 0 |
| 105 | 0 | 9 | 3 | 0 | 0 | 0 |
| 106 | 0 | 9 | 1 | 0 | 9 | 1 |
| 107 | 0 | 9 | 2 | 0 | 9 | 0 |
| 108 | 0 | 0 | | 0 | | 4 |
| 109 | 0 | 9 | 8 | 0 | 9 | 0 |
| 110 | 0 | 9 | 7 | 0 | 9 | 0 |
| 111 | 0 | 9 | 9 | 4 | 9 | 9 |
| 112 | 0 | 9 | 9 | 0 | 9 | 3 |
| 113 | 0 | 9 | 4 | 0 | 9 | 4 |
| 114 | 0 | 9 | 2 | 0 | 9 | 2 |
| 115 | 0 | 9 | 9 | 0 | 9 | 3 |
| 116 | 0 | 9 | 9 | 0 | 8 | 3 |
| 117 | 0 | 9 | 7 | 0 | 8 | 0 |
| 118 | 0 | 7 | 0 | 0 | 8 | 0 |
| 119 | 0 | 8 | 9 | 0 | 0 | 0 |
| 120 | 0 | 9 | 9 | 0 | 9 | 0 |
| 121 | 0 | 9 | 9 | 0 | 9 | 3 |
| 122 | 0 | 9 | 8 | 0 | 0 | 0 |
| 123 | 0 | 9 | 9 | 0 | 0 | 0 |
| 124 | 0 | 5 | | 0 | 0 | 0 |
| 125 | 0 | 9 | 7 | 0 | 8 | 0 |
| 126 | 0 | 9 | 0 | 0 | 9 | 0 |
| 127 | 0 | 9 | 6 | 0 | 8 | 3 |
| 128 | 0 | 9 | 6 | 0 | 9 | 3 |
| 129 | 0 | 9 | 9 | 0 | | 2 |
| 130 | 0 | 9 | 9 | 0 | | 0 |
| 131 | 0 | 9 | 9 | 0 | | 0 |
| 132 | 0 | 9 | 9 | 0 | | 0 |
| 133 | 0 | 9 | 8 | 0 | | 0 |
| 134 | 7 | 9 | 6 | 2 | | 1 |
| 135 | 0 | 9 | | 0 | | 1 |
| 136 | 0 | 9 | | 0 | | 2 |
| 137 | 0 | 9 | | 3 | | 0 |
| 138 | 0 | 6 | | 0 | | 9 |
| 139 | 0 | 8 | | 0 | | 2 |
| 140 | 0 | 9 | | 0 | | 3 |
| 141 | 0 | 8 | 0 | 0 | 9 | 0 |
| 142 | 0 | 2 | | 0 | 0 | 1 |
| 143 | 0 | 0 | | 0 | 0 | 0 |
| 144 | 0 | 2 | | 0 | 9 | 2 |
| 145 | 0 | 9 | 0 | 0 | 0 | 6 |
| 146 | 0 | 9 | 8 | 0 | 9 | 0 |
| 147 | 0 | 9 | | 0 | 0 | 6 |
| 148 | 0 | 6 | | 0 | 0 | 7 |
| 149 | 0 | 9 | | 0 | 9 | 0 |
| 150 | 0 | 0 | | 0 | 0 | 0 |
| 151 | 0 | 0 | | 0 | 9 | 0 |
| 152 | 0 | 4 | 0 | 0 | 9 | 0 |
| 153 | 0 | 0 | | 0 | 0 | 0 |
| 154 | 0 | 9 | 4 | 0 | 8 | 0 |
| 155 | 0 | 9 | 0 | 0 | 9 | 0 |
| 156 | 0 | 0 | | 0 | 9 | 0 |
| 157 | 0 | 9 | 9 | 0 | 9 | 4 |
| 158 | 0 | 8 | 0 | 0 | 9 | 0 |
| 159 | 0 | 9 | 9 | 0 | 9 | 0 |
| 160 | 0 | 4 | | 0 | 9 | 3 |
| 161 | 0 | 9 | 7 | 0 | 0 | 0 |
| 162 | 0 | 9 | 9 | 0 | 8 | 0 |
| 163 | 0 | 6 | | 0 | 8 | 0 |
| 164 | 0 | 9 | 7 | 0 | | 3 |
| 165 | 0 | 9 | | 0 | | 8 |
| 166 | 0 | 9 | 9 | 0 | 9 | 0 |
| 167 | 0 | 9 | 9 | 0 | 7 | 4 |
| 168 | 0 | 9 | 9 | 0 | 9 | 0 |
| 169 | 0 | 9 | 9 | 0 | 9 | 0 |
| 172 | 0 | 9 | 9 | 0 | | 0 |
| 173 | 0 | 9 | 9 | 0 | | 0 |

| | | | | | | |
|---|---|---|---|---|---|---|
| ¹ rates in ppm | | | | | | |

## Claims

1. A method for the control of insect or acarid pests which comprises contacting said pests or their food supply, habitat or breeding grounds with a pesticidally effective amount of a compound having the structural formula 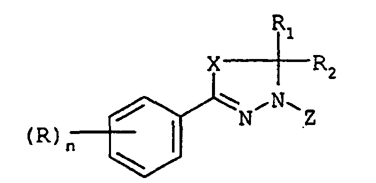 wherein
X is O or S (O)ₘ;
Z is 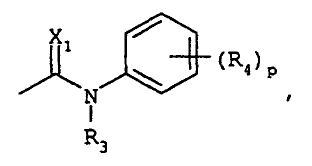
C(X₁)R₅, C₁-C₆alkyl, C₁-C₆haloalkyl,
benzyl optionally substituted on the phenyl ring with any combination of from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups, or
phenyl optionally substituted with any combination of from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups, provided that when X is O, Z is 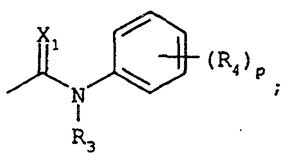
n and p are each independently 0, 1, 2 or 3;
X₁ is O or S;
R and R₄ are each independently halogen, C₁-C₆alkyl, C₁-C₆haloalkyl, OR₆, S(O)_{q}R₇, nitro, cyano,
NR₈R₉, CO₂R₁₀, C(O)R₁₁ or
phenyl optionally substituted with any combination of from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups, or two adjacent R groups or R₄ groups may be taken together to form a ring wherein RR or R₄R₄ is represented by: -OCH₂O-, -OCF₂O- or -CH=CH-CH=CH-;
R₆ and R₇ are each independently hydrogen, C₁-C₆alkyl, C₁-C₆haloalkyl or
phenyl optionally substituted with any combination of from one to three halogen, C₁-C₆alkyl, C₁-C₆haloalkyl, C₁-C₆alkoxy, C₁-C₄haloalkoxy, C₁-C₆alkylthio or C₁-C₆haloalkylthio groups;
R₈, R₉, R₁₃ and R₁₄ are each independently hydrogen, C₁-C₆alkyl, C₁-C₆alkylcarbonyl or
phenyl optionally substituted with any combination of from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups;
R₁₀ and R₁₁ are each independently hydrogen, C₁-C₆alkyl or C₁-C₆haloalkyl;
R₁ and R₂ are each independently hydrogen, C₃-C₇cycloalkyl, C₁-C₆haloalkyl, C₃-C₆alkenyl, C₃-C₆haloalkenyl,
C₃-C₆alkynyl, C₃-C₆haloalkynyl, C₂-C₆alkoxyalkyl, (CH₂)ᵥC(O)R₁₂,
C₁-C₆alkyl optionally substituted with one phenoxy or phenyl group wherein the phenyl ring of each group is independently, optionally substituted with from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups, phenyl optionally substituted with from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups, or
a 5- or 6-membered heteroaromatic ring optionally substituted with any combination of from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups, and
when R₁ and R₂ are taken together with the atom to which they are attached they may form a C₃-C₆cycloalkyl ring wherein R₁R₂ is represented by: -(CH₂)ₜ-where t is 2, 3, 4 or 5;
m, q and v are each independently 0, 1 or 2;
R₁₂ is hydrogen, C₁-C₆alkyl, C₁-C₆haloalkyl, C₁-C₆alkoxy, C₁-C₆haloalkoxy, C₁-C₆alkylthio, C₁-C₆haloalkylthio or NR₁₃R₁₄;
R₃ is hydrogen, C₁-C₆alkyl, C₁-C₆haloalkyl or C(O)R₁₅;
R₁₅ is C₁-C₆alkyl, C₁-C₆haloalkyl, C₁-C₆alkoxy or C₁-C₆haloalkoxy; and
R₅ is C₁-C₆alkyl,
phenyl optionally substituted with any combination of from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups, or benzyl optionally substituted on the phenyl ring with any combination of from one to three halogen, C₁-C₆alkyl, C₁-C₆haloalkyl, C₁-C₆alkoxy, C₁-C₆haloalkoxy, C₁-C₆alkylthio or C₁-C₆haloalkylthio groups; and
the optical isomers thereof and the agriculturally acceptable salts thereof.

2. The method according to claim 1 wherein the compound is selected from the group consisting of
2-(*p*-chlorophenyl)-5,5-dimethyl-4'-(trifluoromethoxy)-Δ²-1,3,4-oxadiazoline-4-carboxanilide;
2-(*p*-chlorophenyl)-5,5-dimethyl-4'-(trifluoromethyl)-Δ²-1,3,4-oxadiazoline-4-carboxanilide;
2-(*p*-bromophenyl)-5,5-dimethyl-4'-(trifluoromethyl)-Δ²-1,3,4-oxadiazoline-4-carboxanilide;
2-(*p*-fluorophenyl)-5,5-dimethyl-4'-(trifluoromethyl)-Δ²-1,3,4-oxadiazoline-4-carboxanilide;
5,5-dimethyl-2-(*p*-phenoxyphenyl)-4'-[(trifluoromethyl)thio]-Δ²-1,3,4-oxadiazoline-4-carboxanilide;
2-(*p*-chlorophenyl)-5-methyl-4'-(trifluoromethoxy)-5-(trifluoromethyl)-Δ²-1,3,4-oxadiazoline-4-carboxanilide;
5-(chloromethyl)-2-(p-chlorophenyl)-5-methyl-4'-(trifluoromethyl)-Δ²-1,3,4-oxadiazoline-4-carboxanilide; 4',5-bis(trifluoromethyl)-2-(p-fluorophenyl)-5-methyl-Δ²-1,3,4-oxadiazoline-4-carboxanilide; 5-(chloromethyl)-2-(p-fluorophenyl)-5-methyl-4'-(trifluoromethyl)-Δ²-1,3,4-oxadiazoline-4-carboxanilide; 5-(chloromethyl)-2-(p-fluorophenyl)-5-methyl-4'-(trifluoromethoxy)-Δ²-1,3,4-oxadiazoline-4-carboxanilide; 2-(p-bromophenyl)-5-(chloromethyl)-5-methyl-4'-(trifluoromethoxy)-Δ²-1,3,4-oxadiazoline-4-carboxanilide; 2-(p-chlorophenyl)-5-methyl-5-(2,2,2-trifluoroethyl)-4'-(trifluoromethyl)-Δ²-1,3,4-oxadiazoline-4-carboxanilide; 2-(p-chlorophenyl)-5-methyl-5-(2,2,2-trifluoroethyl)-4'-(trifluoromethoxy)-Δ²-1,3,4-oxadiazoline-4-carboxanilide; 2-(p-chlorophenyl)-5-methyl-5-(2-pyridyl)-4'-(trifluoromethyl)-Δ²-1,3,4-oxadiazoline-4-carboxanilide; 2-(p-chlorophenyl)-5-methyl-5-(2-pyridyl)-4'-(trifluoromethoxy)-Δ²-1,3,4-oxadiazoline-4-carboxanilide; methyl N-{[2-(p-chlorophenyl)-5,5-dimethyl-Δ²-1,3,4-oxadiazolin-4-yl]carbonyl}-p-(trifluoromethoxy)-carbanilate;
methyl N-{[2-(p-chlorophenyl)-5,5-dimethyl-Δ²-1,3,4-oxadiazolin-4-yl]carbonyl}-p-(trifluoromethyl)-carbanilate; and
methyl 2-(p-chlorophenyl)-5-methyl-4-{[p-(trifluoromethoxy)phenyl]carbamoyl}-Δ²-1,3,4-oxadiazoline-5-acetate.

3. A method for the protection of growing plants from attack or infestation by insect or acarid pests which comprises applying to the foliage of the plants, or to the soil or water in which they are growing, a pesticidally effective amount of a compound having the structural formula 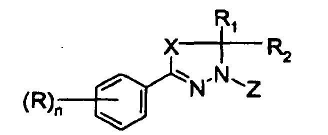 wherein n, R, R₁, R₂, X and Z are as described in claim 1.

4. The method according to claim 3 wherein the compound is selected from the group consisting of 2-(p-chlorophenyl)-5,5-dimethyl-4'-(trifluoromethoxy)-Δ²-1,3,4-oxadiazoline-4-carboxanilide; 2-(p-chlorophenyl)-5,5-dimethyl-4'-(trifluoromethyl)-Δ²-1,3,4-oxadiazoline-4-carboxanilide; 2-(p-bromophenyl)-5,5-dimethyl-4'-(trifluoromethyl)-Δ²-1,3,4-oxadiazoline-4-carboxanilide; 2-(p-fluorophenyl)-5,5-dimethyl-4'-(trifluoromethyl)-Δ²-1,3,4-oxadiazoline-4-carboxanilide; 5,5-dimethyl-2-(p-phenoxyphenyl)-4'-[(trifluoromethyl)thio]-Δ²-1,3,4-oxadiazoline-4-carboxanilide; 2-(p-chlorophenyl)-5-methyl-4'-(trifluoromethoxy)-5-(trifluoromethyl)-Δ²-1,3,4-oxadiazoline-4-carboxanilide; 5-(chloromethyl)-2-(p-chlorophenyl)-5-methyl-4'-(trifluoromethyl)-Δ²-1,3,4-oxadiazoline-4-carboxanilide; 4',5-bis(trifluoromethyl)-2-(p-fluorophenyl)-5-methyl-Δ²-1,3,4-oxadiazoline-4-carboxanilide; 5-(chloromethyl)-2-(p-fluorophenyl)-5-methyl-4'-(trifluoromethyl)-Δ²-1,3,4-oxadiazoline-4-carboxanilide; 5-(chloromethyl)-2-(p-fluorophenyl)-5-methyl-4'-(trifluoromethoxy)-Δ²-1,3,4-oxadiazoline-4-carboxanilide; 2-(p-bromophenyl)-5-(chloromethyl)-5-methyl-4'-(trifluoromethoxy)-Δ²-1,3,4-oxadiazoline-4-carboxanilide; 2-(p-chlorophenyl)-5-methyl-5-(2,2,2-trifluoroethyl)-4'-(trifluoromethyl)-Δ²-1,3,4-oxadiazoline-4-carboxanilide; 2-(p-chlorophenyl)-5-methyl-5-(2,2,2-trifluoroethyl)-4'-(trifluoromethoxy)-Δ²-1,3,4-oxadiazoline-4-carboxanilide; 2-(p-chlorophenyl)-5-methyl-5-(2-pyridyl)-4'-(trifluoromethyl)-Δ²-1,3,4-oxadiazoline-4-carboxanilide; 2-(p-chlorophenyl)-5-methyl-5-(2-pyridyl)-4'-(trifluoromethoxy)-Δ²-1,3,4-oxadiazoline-4-carboxanilide; methyl N-{[2-(p-chlorophenyl)-5,5-dimethyl-Δ²-1,3,4-oxadiazolin-4-yl]carbonyl}-p-(trifluoromethoxy)-carbanilate; methyl N-{[2-(p-chlorophenyl)-5,5-dimethyl-Δ²-1,3,4-oxadiazolin-4-yl]carbonyl}-p-(trifluoromethyl)-carbanilate; and methyl 2-(p-chlorophenyl)-5-methyl-4-{[p-(trifluoromethoxy)phenyl]carbamoyl}-Δ²-1,3,4-oxadiazoline-5-acetate.

5. The method according to claim 3 wherein the compound is applied to the plants, or to the soil or water in which they are growing, at a rate of 0.1 kg/ha to 4.0 kg/ha.

6. A compound having the structural formula II 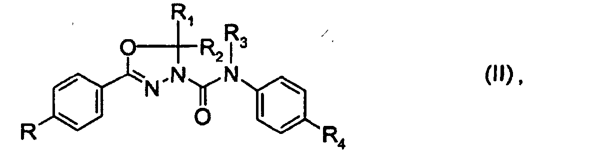 wherein
R is halogen, C₁-C₄-haloalkyl, C₁-C₄-haloalkoxy or phenoxy optionally substituted with any combination of from one to three halogen, C₁-C₄-alkyl, C₁-C₄-haloalkyl, C₁-C₄-alkoxy or C₁-C₄-haloalkoxy groups;
R₄ is C₁-C₄-haloalkyl, C₁-C₄-haloalkoxy or C₁-C₄-haloalkylthio;
R₁ is C₁-C₄-alkyl;
R₂ is C₁-C₄-alkyl, C₁-C₄-haloalkyl, (CH₂)ᵥC(O)R₁₂ or 2-pyridyl optionally substituted with any combination of from one to three halogen, C₁-C₄-alkyl, C₁-C₄-haloalkyl, C₁-C₄-alkoxy or C₁-C₄-haloalkoxy groups;
v is 0 or 1;
R₁₂ is C₁-C₄-alkoxy or C₁-C₄-haloalkoxy;
R₃ is hydrogen or C(O)R₁₅; and
R₁₅ is C₁-C₄-alkoxy.

7. A compound selected from the group consisting of 2-(p-chlorophenyl)-5,5-dimethyl-4'-(trifluoromethoxy)-Δ²-1,3,4-oxadiazoline-4-carboxanilide; 2-(p-chlorophenyl)-5,5-dimethyl-4'-(trifluoromethyl)-Δ²-1,3,4-oxadiazoline-4-carboxanilide; 2-(p-bromophenyl)-5,5-dimethyl-4'-(trifluoromethyl)-Δ²-1,3,4-oxadiazoline-4-carboxanilide; 2-(p-fluorophenyl)-5,5-dimethyl-4'-(trifluoromethyl)-Δ²-1,3,4-oxadiazoline-4-carboxanilide; 5,5-dimethyl-2-(p-phenoxyphenyl)-4'-[(trifluoromethyl)thio]-Δ²-1,3,4-oxadiazoline-4-carboxanilide; 2-(p-chlorophenyl)-5-methyl-4'-(trifluoromethoxy)-5-(trifluoromethyl)-Δ²-1,3,4-oxadiazoline-4-carboxanilide; 5-(chloromethyl)-2-(p-chlorophenyl)-5-methyl-4'-(trifluoromethyl)-Δ²-1,3,4-oxadiazoline-4-carboxanilide; 4',5-bis(trifluoromethyl)-2-(p-fluorophenyl)-5-methyl-Δ²-1,3,4-oxadiazoline-4-carboxanilide; 5-(chloromethyl)-2-(p-fluorophenyl)-5-methyl-4'-(trifluoromethyl)-Δ²-1,3,4-oxadiazoline-4-carboxanilide; 5-(chloromethyl)-2-(p-fluorophenyl)-5-methyl-4'-(trifluoromethoxy)-Δ²-1,3,4-oxadiazoline-4-carboxanilide; 2-(p-bromophenyl)-5-(chloromethyl)-5-methyl-4'-(trifluoromethoxy)-Δ²-1,3,4-oxadiazoline-4-carboxanilide; 2-(p-chlorophenyl)-5-methyl-5-(2,2,2-trifluoroethyl)-4'-(trifluoromethyl)-Δ²-1,3,4-oxadiazoline-4-carboxanilide; 2-(p-chlorophenyl)-5-methyl-5-(2,2,2-trifluoroethyl)-4'-(trifluoromethoxy)-Δ²-1,3,4-oxadiazoline-4-carboxanilide; 2-(p-chlorophenyl)-5-methyl-5-(2-pyridyl)-4'-(trifluoromethyl)-Δ²-1,3,4-oxadiazoline-4-carboxanilide; 2-(p-chlorophenyl)-5-methyl-5-(2-pyridyl)-4'-(trifluoromethoxy)-Δ²-1,3,4-oxadiazoline-4-carboxanilide; methyl N-{[2-(p-chlorophenyl)-5,5-dimethyl-Δ²-1,3,4-oxadiazolin-4-yl]carbonyl}-p-(trifluoromethoxy)-carbanilate; methyl N-{[2-(p-chlorophenyl)-5,5-dimethyl-Δ²-1,3,4-oxadiazolin-4-yl]carbonyl}-p-(trifluoromethyl)-carbanilate; and methyl 2-(p-chlorophenyl)-5-methyl-4-{[p-(trifluoromethoxy)phenyl]carbamoyl}-Δ²-1,3,4-oxadiazoline-5-acetate.

8. A composition for the control of insect or acarid pests which comprises an agronomically acceptable carrier and a pesticidally effective amount of a compound having the structural formula II 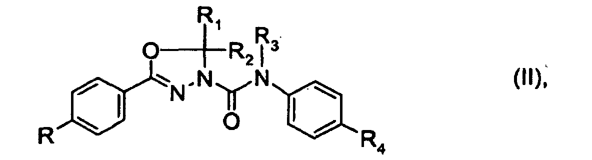 wherein
R is halogen, C₁-C₄-haloalkyl, C₁-C₄-haloalkoxy or phenoxy optionally substituted with any combination of from one to three halogen, C₁-C₄-alkyl, C₁-C₄-haloalkyl, C₁-C₄-alkoxy or C₁-C₄-haloalkoxy groups;
R₄ is C₁-C₄-haloalkyl, C₁-C₄-haloalkoxy or C₁-C₄-haloalkylthio;
R₁ is C₁-C₄-alkyl;
R₂ is C₁-C₄-alkyl, C₁-C₄-haloalkyl, (CH₂)ᵥC(O)R₁₂ or 2-pyridyl optionally substituted with any combination of from one to three halogen, C₁-C₄-alkyl, C₁-C₄-
haloalkyl, C₁-C₄-alkoxy or C₁-C₄-haloalkoxy groups;
v is 0 or 1;
R₁₂ is C₁-C₄-alkoxy or C₁-C₄-haloalkoxy;
R₃ is hydrogen or C(O)R₁₅; and
R₁₅ is C₁-C₄-alkoxy.

9. The composition according to claim 8 wherein the compound is selected from the group consisting of 2-(p-chlorophenyl)-5,5-dimethyl-4'-(trifluoromethoxy)-Δ²-1,3,4-oxadiazoline-4-carboxanilide; 2-(p-chlorophenyl)-5,5-dimethyl-4'-(trifluoromethyl)-Δ²-1,3,4-oxadiazoline-4-carboxanilide; 2-(p-bromophenyl)-5,5-dimethyl-4'-(trifluoromethyl)-Δ²-1,3,4-oxadiazoline-4-carboxanilide; 2-(p-fluorophenyl)-5,5-dimethyl-4'-(trifluoromethyl)-Δ²-1,3,4-oxadiazoline-4-carboxanilide; 5,5-dimethyl-2-(p-phenoxyphenyl)-4'-[(trifluoromethyl)thio]-Δ²-1,3,4-oxadiazoline-4-carboxanilide; 2-(p-chlorophenyl)-5-methyl-4'-(trifluoromethoxy)-5-(trifluoromethyl)-Δ²-1,3,4-oxadiazoline-4-carboxanilide; 5-(chloromethyl)-2-(p-chlorophenyl)-5-methyl-4'-(trifluoromethyl)-Δ²-1,3,4-oxadiazoline-4-carboxanilide; 4',5-bis(trifluoromethyl)-2-(p-fluorophenyl)-5-methyl-Δ²-1,3,4-oxadiazoline-4-carboxanilide; 5-(chloromethyl)-2-(p-fluorophenyl)-5-methyl-4'-(trifluoromethyl)-Δ²-1,3,4-oxadiazoline-4-carboxanilide; 5-(chloromethyl)-2-(p-fluorophenyl)-5-methyl-4'-(trifluoromethoxy)-Δ²-1,3,4-oxadiazoline-4-carboxanilide; 2-(p-bromophenyl)-5-(chloromethyl)-5-methyl-4'-(trifluoromethoxy)-Δ²-1,3,4-oxadiazoline-4-carboxanilide; 2-(p-chlorophenyl)-5-methyl-5-(2,2,2-trifluoroethyl)-4'-(trifluoromethyl)-Δ²-1,3,4-oxadiazoline-4-carboxanilide; 2-(p-chlorophenyl)-5-methyl-5-(2,2,2-trifluoroethyl)-4'-(trifluoromethoxy)-Δ²-1,3,4-oxadiazoline-4-carboxanilide; 2-(p-chlorophenyl)-5-methyl-5-(2-pyridyl)-4'-(trifluoromethyl)-Δ²-1,3,4-oxadiazoline-4-carboxanilide; 2-(p-chlorophenyl)-5-methyl-5-(2-pyridyl)-4'-(trifluoromethoxy)-Δ²-1,3,4-oxadiazoline-4-carboxanilide; methyl N-{[2-(p-chlorophenyl)-5,5-dimethyl-Δ²-1,3,4-oxadiazolin-4-yl]carbonyl}-p-(trifluoromethoxy)-carbanilate; methyl N-{[2-(p-chlorophenyl)-5,5-dimethyl-Δ²-1,3,4-oxadiazolin-4-yl]carbonyl}-p-(trifluoromethyl)-carbanilate; and methyl 2-(p-chlorophenyl)-5-methyl-4-{[p-(trifluoromethoxy)phenyl]carbamoyl}-Δ²-1,3,4-oxadiazoline-5-acetate.

## Patentansprüche

1. Verfahren zur Bekämpfung von Schadinsekten oder Schadakariden, **dadurch gekennzeichnet, daß** man diese Schädlinge oder ihre Nahrung, Umwelt oder Brutstätten mit einer pestizid wirksamen Menge einer Verbindung der Strukturformel 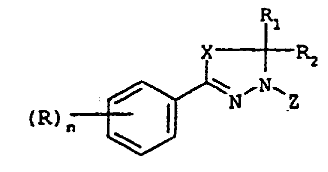 in der X O oder S(O)ₘ bedeutet; Z 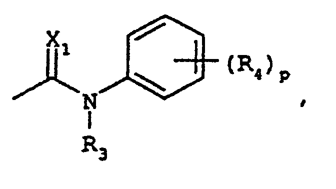 C(X₁)R₅, C₁-C₆-Alkyl, C₁-C₆-Halogenalkyl,
Benzyl, das gegebenenfalls am Phenylring mit einer beliebigen Kombination von einer bis drei Halogen-, C₁-C₆-Alkyl-, C₁-C₆-Halogenalkyl-, C₁-C₆-Alkoxy-, C₁-C₆-Halogenalkoxy-, C₁-C₆-Alkylthio- oder C₁C₆-Halogenalkylthiogruppen substituiert, oder
Phenyl, das gegebenenfalls mit einer beliebigen Kombination von einer bis drei Halogen-, C₁-C₆-Alkyl-, C₁-C₆-Halogenalkyl, C₁-C₆-Alkoxy, C₁-C₆-Halogenalkoxy-, C₁-C₆-Alkylthio- oder C₁-C₆-Halogenalkylthiogruppen substituiert ist,
bedeutet, mit der Maßgabe, daß, wenn X O bedeutet, Z 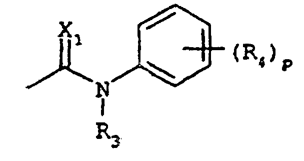 bedeutet;
n und p jeweils unabhängig 0, 1, 2 oder 3 bedeuten;
X₁ O oder S bedeutet;
R und R₄ jeweils unabhängig Halogen, C₁-C₆-Alkyl, C₁-C₆-Halogenalkyl, OR₆, S(O)_{q}R₇, Nitro, Cyano, NR₈R₉, CO₂R₁₀, C(O)R₁₁ oder
Phenyl, das gegebenenfalls mit einer beliebigen Kombination von einer bis drei Halogen-, C₁-C₆-Alkyl, C₁-C₆-Halogenalkyl, C₁-C₆-Alkoxy, C₁-C₆-Halogenalkoxy, C₁-C₆-Alkylthio oder C₁-C₆-Halogenalkylthiogruppen substituiert ist, bedeuten, oder
zwei benachbarte Gruppen R oder R₄ können gemeinsam einen Ring bilden, wobei RR oder R₄R₄ -OCH₂O-, -OCF₂Ooder -CH=CH-CH=CH- entsprechen;
R₆ und R₇ jeweils unabhängig Wasserstoff, C₁-C₆-Alkyl, C₁-C₆-Halogenalkyl oder
Phenyl, das gegebenenfalls mit einer beliebigen Kombination von einer bis drei Halogen-, C₁-C₆Alkyl-, C₁-C₆-Halogenalkyl-, C₁-C₆-Alkoxy-, C₁-C₄-Halogenalkoxy-, C₁-C₆-Alkylthio- oder C₁-C₆-Halogenalkylthiogruppen substituiert ist, bedeuten;
R₈, R₉ R₁₃ und R₁₄ jeweils unabhängig Wasserstoff, C₁-C₆-Alkyl, C₁-C₆-Alkylcarbonyl oder
Phenyl, das gegebenenfalls mit einer beliebigen Kombination von einer bis drei Halogen-, C₁-C₆-Alkyl-, C₁-C₆-Halogenalkyl-, C₁-C₆-Alkoxy-, C₁-C₄-Halogenalkoxy-, C₁-C₆-Alkylthio- oder C₁-C₆-Halogenalkylthiogruppen substituiert ist, bedeuten;
R₁₀ und R₁₁ jeweils unabhängig Wasserstoff, C₁-C₆-Alkyl oder C₁-C₆-Halogenalkyl bedeuten;
R₁ und R₂ jeweils unabhängig Wasserstoff, C₃-C₇-Cycloalkyl, C₁-C₆-Halogenalkyl, C₃-C₆-Alkenyl, C₃-C₆-Halogenalkenyl, C₃-C₆-Alkinyl, C₃-C₆-Halogenalkinyl, C₂-C₆-Alkoxyalkyl, (CH₂)ᵥC(O)R₁₂,
C₁-C₆-Alkyl, das gegebenenfalls mit einer Phenoxy- oder Phenylgruppe substituiert ist, wobei der Phenylring jeder Gruppe unabhängig gegebenenfalls mit einer bis drei Halogen-, C₁-C₆-Alkyl-, C₁-C₆-Halogenalkyl, C₁-C₆-Alkoxy, C₁-C₆-Halogenalkoxy-, C₁-C₆-Alkylthio- oder C₁-C₆-Halogenalkylthiogruppen substituiert ist,
Phenyl, das gegebenenfalls mit einer bis drei Halogen-, C₁-C₆-Alkyl-, C₁-C₆-Halogenalkyl-, C₁-C₆-Alkoxy-, C₁-C₆-Halogenalkoxy-, C₁-C₆-Alkylthio- oder C₁-C₆-Halogenalkylthiogruppen substituiert ist, oder
einen 5- oder 6-gliedrigen Heterocyclus, der gegebenenfalls mit einer beliebigen Kombination von einer bis drei Halogen-, C₁-C₆-Alkyl-, C₁-C₆-Halogenalkyl-, C₁-C₆-Alkoxy-, C₁-C₆-Halogenalkoxy-, C₁-C₆-Alkylthio- oder C₁-C₆Halogenalkylthiogruppen substituiert ist,
bedeuten, und,
R₁ und R₂ gemeinsam mit dem Atom, an das sie gebunden sind, einen C₃-C₆-Cycloalkylring bilden können, in dem R₁R₂ -(CH₂)ₜ- entspricht, wobei t 2, 3, 4 oder 5 bedeutet;
m, q und v jeweils unabhängig 0, 1 oder 2 bedeuten;
R₁₂ Wasserstoff, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio oder NR₁₃R₁₄ bedeutet;
R₃ Wasserstoff, C₁-C₆-Alkyl, C₁-C₆-Halogenalkyl oder C(O)R₁₅ bedeutet;
R₁₅ C₁-C₆-Alkyl, C₁-C₆-Halogenalkyl, C₁-C₆-Alkoxy oder C₁-C₆-Halogenalkoxy bedeutet; und
R₅ C₁-C₆-Alkyl,
Phenyl, das gegebenenfalls mit einer beliebigen Kombination von einer bis drei Halogen-, C₁-C₆-Alkyl-, C₁-C₆-Halogenalkyl-, C₁-C₆-Alkoxy-, C₁-C₆-Halogenalkoxy-, C₁-C₆-Alkylthio- oder C₁-C₆-Halogenalkylthiogruppen substituiert ist, oder
Benzyl, das gegebenenfalls am Phenylring mit einer beliebigen Kombination von einer bis drei Halogen-, C₁-C₆-Alkyl-, C₁-C₆-Halogenalkyl-, C₁-C₆-Alkoxy-, C₁-C₆-Halogenalkoxy-, C₁-C₆-Alkylthio- oder C₁-C₆-Halogenalkylthiogruppen substituiert ist,
bedeutet; und
ihren optischen Isomeren und ihren landwirtschaftlich verträglichen Salzen in Kontakt bringt.

2. Verfahren nach Anspruch 1, **dadurch gekennzeichnet, daß** die Verbindung aus der Gruppe
2-(*p*-Chlorphenyl)-5,5-dimethyl-4'-(trifluormethoxy)-Δ²-1,3,4-oxadiazolin-4-carboxanilid,
2-(*p*-Chlorphenyl)-5,5-dimethyl-4'-(trifluormethyl)-Δ²-1,3,4-oxadiazolin-4-carboxanilid,
2-(*p*-Bromphenyl)-5,5-dimethyl-4'-(trifluormethyl)-Δ²-1,3,4-oxadiazolin-4-carboxanilid,
2-(*p*-Fluorphenyl)-5,5-dimethyl-4'-(trifluormethyl)-Δ²-1,3,4-oxadiazolin-4-carboxanilid,
5,5-Dimethyl-2-(*p*-phenoxyphenyl)-4'-[(trifluormethyl)-thio]-Δ²-1,3,4-oxadiazolin-4-carboxanilid,
2-(*p*-Chlorphenyl)-5-methyl-4'-(trifluormethoxy)-5-(trifluormethyl)-Δ²-1,3,4-oxadiazolin-4-carboxanilid,
5-(Chlormethyl)-2-(*p*-chlorphenyl)-5-methyl-4'-(trifluormethyl)-Δ²-1,3,4-oxadiazolin-4-carboxanilid,
4',5-Bis(trifluormethyl)-2-(*p*-fluorphenyl)-5-methyl-Δ²-1,3,4-oxadiazolin-4-carboxanilid,
5-(Chlormethyl)-2-(*p*-fluorphenyl)-5-methyl-4'-(trifluormethyl)-Δ²-1,3,4-oxadiazolin-4-carboxanilid,
5-(Chlormethyl)-2-(*p*-fluorphenyl)-5-methyl-4'-(trifluormethoxy)-Δ²-1,3,4-oxadiazolin-4-carboxanilid,
2-(*p*-Bromphenyl)-5-(chlormethyl)-5-methyl-4'-(trifluormethoxy)-Δ²-1,3,4-oxadiazolin-4-carboxanilid,
2-(*p*-Chlorphenyl)-5-methyl-5-(2,2,2-trifluorethyl)-4'-(trifluormethyl)-Δ²-1,3,4-oxadiazolin-4-carboxanilid,
2-(*p*-Chlorphenyl)-5-methyl-5-(2,2,2-trifluorethyl)-4'-(trifluormethoxy)-Δ²-1,3,4-oxadiazolin-4-carboxanilid,
2-(*p*-Chlorphenyl)-5-methyl-5-(2-pyridyl)-4'-(trifluormethyl)-Δ²-1,3,4-oxadiazolin-4-carboxanilid,
2-(*p*-Chlorphenyl)-5-methyl-5-(2-pyridyl)-4'-(trifluormethoxy)-Δ²-1,3,4-oxadiazolin-4-carboxanilid,
Methyl-N-{[2-(*p*-chlorphenyl)-5,5-dimethyl-Δ²-1,3,4-oxadiazolin-4-yl]carbonyl}-*p*-(trifluormethoxy)carbanilat,
Methyl-N-{[2-(*p*-chlorphenyl)-5,5-dimethyl-Δ²-1,3,4-oxadiazolin-4-yl]carbonyl}-*p*-(trifluormethyl)carbanilat und
Methyl-2-(*p*-chlorphenyl)-5-methyl-4-{[p-(trifluormethoxy)phenyl]carbamoyl}-Δ²-1,3,4-oxadiazolin-5-acetat
stammt.

3. Verfahren zum Schutz wachsender Pflanzen gegen Angriff oder Befall durch Schadinsekten oder Schadakariden, **dadurch gekennzeichnet, daß** man auf das Blattwerk der Pflanzen oder auf den Boden bzw. das Wasser in dem sie wachsen, eine pestizid wirksame Menge einer Verbindung der Strukturformel 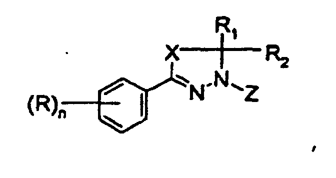 in der n, R, R₁, R₂, X und Z wie in Anspruch 1 definiert sind,
aufbringt.

4. Verfahren nach Anspruch 3, wobei die Verbindung aus der Gruppe
2-(*p*-Chlorphenyl)-5,5-dimethyl-4'-(trifluormethoxy)-Δ²-1,3,4-oxadiazolin-4-carboxanilid,
2-(*p*-Chlorphenyl)-5,5-dimethyl-4'-(trifluormethyl)-Δ²-1,3,4-oxadiazolin-4-carboxanilid,
2-(*p*-Bromphenyl)-5,5-dimethyl-4'-(trifluormethyl)-Δ²-1,3,4-oxadiazolin-4-carboxanilid,
2-(*p*-Fluorphenyl)-5,5-dimethyl-4'-(trifluormethyl)-Δ²-1,3,4-oxadiazolin-4-carboxanilid,
5,5-Dimethyl-2-(*p*-phenoxyphenyl)-4'-[(trifluormethyl)thio]-Δ²-1,3,4-oxadiazolin-4-carboxanilid,
2-(*p*-Chlorphenyl)-5-methyl-4'-(trifluormethoxy)-5-(trifluormethyl)-Δ²-1,3,4-oxadiazolin-4-carboxanilid,
5-(Chlormethyl)-2-(*p*-chlorphenyl)-5-methyl-4'-(trifluormethyl)-Δ²-1,3,4-oxadiazolin-4-carboxanilid,
4',5-Bis(trifluormethyl)-2-(*p*-fluorphenyl)-5-methyl-Δ²-1,3,4-oxadiazolin-4-carboxanilid,
5-(Chlormethyl)-2-(*p*-fluorphenyl)-5-methyl-4'-(trifluormethyl)-Δ²-1,3,4-oxadiazolin-4-carboxanilid,
5-(Chlormethyl)-2-(*p*-fluorphenyl)-5-methyl-4'-(trifluormethoxy)-Δ²-1,3,4-oxadiazolin-4-carboxanilid,
2-(*p*-Bromphenyl)-5-(chlormethyl)-5-methyl-4'-(trifluormethoxy)-Δ²-1,3,4-oxadiazolin-4-carboxanilid,
2-(*p*-Chlorphenyl)-5-methyl-5-(2,2,2-trifluorethyl)-4'-(trifluormethyl)-Δ²-1,3,4-oxadiazolin-4-carboxanilid,
2-(*p*-Chlorphenyl)-5-methyl-5-(2,2,2-trifluorethyl)-4'-(trifluormethoxy)-Δ²-1,3,4-oxadiazolin-4-carboxanilid,
2-(*p*-Chlorphenyl)-5-methyl-5-(2-pyridyl)-4'-(trifluormethyl)-Δ²-1,3,4-oxadiazolin-4-carboxanilid,
2-(*p*-Chlorphenyl)-5-methyl-5-(2-pyridyl)-4'-(trifluormethoxy)-Δ²-1,3,4-oxadiazolin-4-carboxanilid,
Methyl-N-{[2-(*p*-chlorphenyl)-5,5-dimethyl-Δ²-1,3,4-oxadiazolin-4-yl]carbonyl}-*p*-(trifluormethoxy)carbanilat,
Methyl-N-{[2-(*p*-chlorphenyl)-5,5-dimethyl-Δ²-1,3,4-oxadiazolin-4-yl]carbonyl}-*p*-(trifluormethyl)carbanilat und
Methyl-2-(*p*-chlorphenyl)-5-methyl-4-{[p-(trifluormethoxy)phenyl]carbamoyl}-Δ²-1,3,4-oxadiazolin-5-acetat
stammt.

5. Verfahren nach Anspruch 3, wobei die Verbindung auf die Pflanzen oder den Boden oder das Wasser, in dem sie wachsen, in einer Aufwandmenge von 0,1 kg/ha bis 4,0 kg/ha ausgebracht wird.

6. Verbindung der Strukturformel II 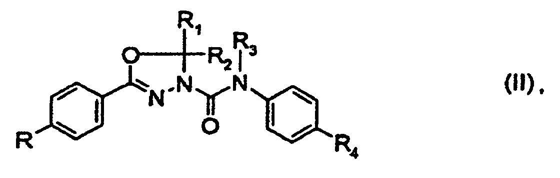 in der
R Halogen, C₁-C₄-Halogenalkyl, C₁-C₄-Halogenalkoxy oder Phenoxy, das gegebenenfalls mit einer beliebigen Kombination von einer bis drei Halogen- C₁-C₄-Alkyl-, C₁-C₄-Halogenalkyl-, C₁-C₄-Alkoxy- oder C₁-C₄-Halogenalkoxygruppen substituiert ist, bedeutet;
R₄ C₁-C₄-Halogenalkyl-, C₁-C₄-Halogenalkoxy oder C₁-C₄-Halogenalkylthio bedeutet;
R₁ C₁-C₄-Alkyl bedeutet;
R₂ C₁-C₄-Alkyl, C₁-C₄-Halogenalkyl, (CH₂)ᵥC(O)R₁₂ oder 2-Pyridyl, das gegebenenfalls mit einer beliebigen Kombination von einer bis drei Halogen-, C₁-C₄-Alkyl-, C₁-C₄-Halogenalkyl-, C₁-C₄-Alkoxy- oder C₁-C₄-Halogenalkoxygruppen substituiert ist, bedeutet;
v 0 oder 1 bedeutet;
R₁₂ C₁-C₄-Alkoxy oder C₁-C₄-Halogenalkoxy bedeutet;
R₃ Wasserstoff oder C(O)R₁₅ bedeutet; und
R₅ C₁-C₄-Alkoxy bedeutet.

7. Verbindung aus der Gruppe
2-(*p*-Chlorphenyl)-5,5-dimethyl-4'-(trifluormethoxy)-Δ²-1,3,4-oxadiazolin-4-carboxanilid,
2-(*p*-Chlorphenyl)-5,5-dimethyl-4'-(trifluormethyl)-Δ²-1,3,4-oxadiazolin-4-carboxanilid,
2-(*p*-Bromphenyl)-5,5-dimethyl-4'-(trifluormethyl)-Δ²-1,3,4-oxadiazolin-4-carboxanilid,
2-(*p*-Fluorphenyl)-5,5-dimethyl-4'-(trifluormethyl)-Δ²-1,3,4-oxadiazolin-4-carboxanilid,
5,5-Dimethyl-2-(*p*-phenoxyphenyl)-4'-[(trifluormethyl)thio]-Δ²-1,3,4-oxadiazolin-4-carboxanilid,
2-(*p*-Chlorphenyl)-5-methyl-4'-(trifluormethoxy)-5-(trifluormethyl)-Δ²-1,3,4-oxadiazolin-4-carboxanilid,
5-(Chlormethyl)-2-(*p*-chlorphenyl)-5-methyl-4'-(trifluormethyl)-Δ²-1,3,4-oxadiazolin-4-carboxanilid,
4',5-Bis(trifluormethyl)-2-(*p*-Fluorphenyl)-5-methyl-Δ²-1,3,4-oxadiazolin-4-carboxanilid,
5-(Chlormethyl)-2-(*p*-fluorphenyl)-5-methyl-4'-(trifluormethyl)-Δ²-1,3,4-oxadiazolin-4-carboxanilid,
5-(Chlormethyl)-2-(*p*-fluorphenyl)-5-methyl-4'-(trifluormethoxy)-Δ²-1,3,4-oxadiazolin-4-carboxanilid,
2-(*p*-Bromphenyl)-5-(chlormethyl)-5-methyl-4'-(trifluormethoxy)-Δ²-1,3,4-oxadiazolin-4-carboxanilid,
2-(*p*-Chlorphenyl)-5-methyl-5-(2,2,2-trifluorethyl)-4'-(trifluormethyl)-Δ²-1,3,4-oxadiazolin-4-carboxanilid,
2-(*p*-Chlorphenyl)-5-methyl-5-(2,2,2-trifluorethyl)-4'-(trifluormethoxy)-Δ²-1,3,4-oxadiazolin-4-carboxanilid,
2-(*p*-Chlorphenyl)-5-methyl-5-(2-pyridyl)-4'-(trifluormethyl)-Δ²-1,3,4-oxadiazolin-4-carboxanilid,
2-(*p*-Chlorphenyl)-5-methyl-5-(2-pyridyl)-4'-(trifluormethoxy)-Δ²-1,3,4-oxadiazolin-4-carboxanilid,
Methyl-N-{[2-(*p*-chlorphenyl)-5,5-dimethyl-Δ²-1,3,4-oxadiazolin-4-yl]carbonyl}-*p*-(trifluormethoxy)carbanilat,
Methyl-N-{[2-(*p*-chlorphenyl)-5,5-dimethyl-Δ²-1,3,4-oxadiazolin-4-yl]carbonyl}-*p*-(trifluormethyl)carbanilat und
Methyl-2-(*p*-chlorphenyl)-5-methyl-4-{[p-(trifluormethoxy)phenyl]carbamoyl}-Δ²-1,3,4-oxadiazolin-5-acetat.

8. Zusammensetzung für die Bekämpfung von Schadinsekten oder Schadakariden, die einen landwirtschaftlich unbedenklichen Träger und eine pestizid wirksame Menge einer Verbindung der Strukturformel II 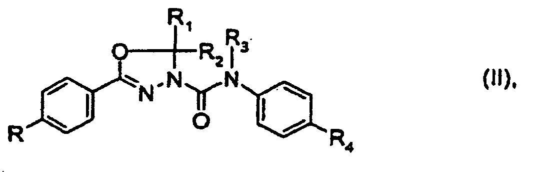 in der
R Halogen, C₁-C₄-Halogenalkyl, C₁-C₄-Halogenalkoxy oder Phenoxy, das gegebenenfalls mit einer beliebigen Kombination von einer bis drei Halogen-, C₁-C₄-Alkyl-, C₁-C₄-Halogenalkyl-, C₁-C₄-Alkoxy- oder C₁-C₄-Halogenalkoxygruppen substituiert ist, bedeutet;
R₄ C₁-C₄-Halogenalkyl-, C₁-C₄-Halogenalkoxy oder C₁-C₄-Halogenalkylthio bedeutet;
R₁ C₁-C₄-Alkyl bedeutet;
R₂ C₁-C₄-Alkyl, C₁-C₄-Halogenalkyl, (CH₂)ᵥC(O)R₁₂ oder 2-Pyridyl, das gegebenenfalls mit einer beliebigen Kombination von einer bis drei Halogen-, C₁-C₄-Alkyl-, C₁-C₄-Halogenalkyl-, C₁-C₄-Alkoxy- oder C₁-C₄-Halogenalkoxygruppen substituiert ist, bedeutet;
v 0 oder 1 bedeutet;
R₁₂ C₁-C₄-Alkoxy oder C₁-C₄-Halogenalkoxy bedeutet;
R₃ Wasserstoff oder C(O)R₁₅ bedeutet; und
R₅ C₁-C₄-Alkoxy bedeutet,
enthält.

9. Zusammensetzung nach Anspruch 8, wobei die Verbindung aus der Gruppe
2-(*p*-Chlorphenyl)-5,5-dimethyl-4'-(trifluormethoxy)-Δ²-1,3,4-oxadiazolin-4-carboxanilid,
2-(*p*-Chlorphenyl)-5,5-dimethyl-4'-(trifluormethyl)-Δ²-1,3,4-oxadiazolin-4-carboxanilid,
2-(*p*-Bromphenyl)-5,5-dimethyl-4'-(trifluormethyl)-Δ²-1,3,4-oxadiazolin-4-carboxanilid,
2-(*p*-Fluorphenyl)-5,5-dimethyl-4'-(trifluormethyl)-Δ²-1,3,4-oxadiazolin-4-carboxanilid,
5,5-Dimethyl-2-(*p*-phenoxyphenyl)-4'-[(trifluormethyl)thio]-Δ²-1,3,4-oxadiazolin-4-carboxanilid,
2-(*p*-Chlorphenyl)-5-methyl-4'-(trifluormethoxy)-5-(trifluormethyl)-Δ²-1,3,4-oxadiazolin-4-carboxanilid,
5-(Chlormethyl)-2-(*p*-chlorphenyl)-5-methyl-4'-(trifluormethyl)-Δ²-1,3,4-oxadiazolin-4-carboxanilid,
4',5-Bis(trifluormethyl)-2-(*p*-Fluorphenyl)-5-methyl-Δ²-1,3,4-oxadiazolin-4-carboxanilid,
5-(Chlormethyl)-2-(*p*-fluorphenyl)-5-methyl-4'-(trifluormethyl)-Δ²-1,3,4-oxadiazolin-4-carboxanilid,
5-(Chlormethyl)-2-(*p*-fluorphenyl)-5-methyl-4'-(trifluormethoxy)-Δ²-1,3,4-oxadiazolin-4-carboxanilid,
2-(*p*-Bromphenyl)-5-(chlormethyl)-5-methyl-4'-(trifluormethoxy)-Δ²-1,3,4-oxadiazolin-4-carboxanilid,
2-(*p*-Chlorphenyl)-5-methyl-5-(2,2,2-trifluorethyl)-4'-(trifluormethyl)-Δ²-1,3,4-oxadiazolin-4-carboxanilid,
2-(*p*-Chlorphenyl)-5-methyl-5-(2,2,2-trifluorethyl)-4'-(trifluormethoxy)-Δ²-1,3,4-oxadiazolin-4-carboxanilid,
2-(*p*-Chlorphenyl)-5-methyl-5-(2-pyridyl)-4'-(trifluormethyl)-Δ²-1,3,4-oxadiazolin-4-carboxanilid,
2-(*p*-Chlorphenyl)-5-methyl-5-(2-pyridyl)-4'-(trifluormethoxy)-Δ²-1,3,4-oxadiazolin-4-carboxanilid,
Methyl-N-{[2-(*p*-chlorphenyl)-5,5-dimethyl-Δ²-1,3,4-oxadiazolin-4-yl]carbonyl}-*p*-(trifluormethoxy)carbanilat,
Methyl-N-{[2-(*p*-chlorphenyl)-5,5-dimethyl-Δ²-1,3,4-oxadiazolin-4-yl]carbonyl}-*p*-(trifluormethyl)carbanilat und
Methyl-2-(*p*-chlorphenyl)-5-methyl-4-{[*p*-(trifluormethoxy)phenyl]carbamoyl}-Δ²-1,3,4-oxadiazolin-5-acetat
stammt.

## Revendications

1. Procédé pour lutter contre des organismes nuisibles, à savoir des insectes ou des acariens, qui comprend la mise en contact desdits organismes nuisibles ou de leur apport nutritif, de leur habitat ou de leurs zones de reproduction avec une quantité efficace du point de vue pesticide d'un composé répondant à la formule développée 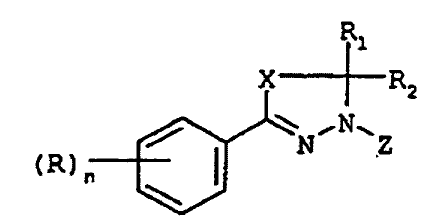 dans laquelle
X représente un atome d'oxygène ou un groupe S(O)ₘ ;
Z représente 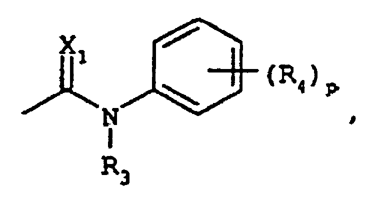 un groupe C(X₁)R₅, un groupe alkyle en C₁-C₆, un groupe halogénalkyle en C₁-C₆, un groupe benzyle le cas échéant substitué sur le noyau phényle avec une quelconque combinaison de un à trois membres choisis parmi le groupe comprenant
un atome d'halogène, un groupe alkyle en C₁-C₆, un groupe halogénalkyle en C₁-C₆, un groupe alcoxy en C₁-C₆, un groupe halogénalcoxy en C₁-C₆, un groupe alkyl(en C₁-C₆)thio ou un groupe halogénalkyl(en C₁-C₆)thio ; ou
un groupe phényle le cas échéant substitué avec une quelconque combinaison de un à trois membres choisis parmi le groupe comprenant un atome d'halogène, un groupe alkyle en C₁-C₆, un groupe halogénalkyle en C₁-C₆, un groupe alcoxy en C₁-C₆, un groupe halogénalcoxy en C₁-C₆, un groupe alkyl(en C₁-C₆)thio ou un groupe halogénalkyl(en C₁-C₆)thio ;
avec cette réserve que lorsque X représente un atome d'oxygène, Z
représente : 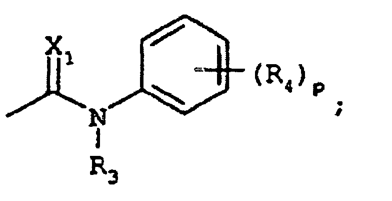 n et p représentent chacun, indépendamment l'un de l'autre, 0, 1, 2 ou 3 ;
X₁ représente un atome d'oxygène ou un atome de soufre ;
R et R₄ représentent chacun, indépendamment l'un de l'autre, un atome d'halogène, un groupe alkyle en C₁-C₆, un groupe halogénalkyle en C₁-C₆, un groupe OR₆, un groupe S(O)_{q}R₇, un groupe nitro, un groupe cyano, un groupe
NR₈R₉, un groupe CO₂R₁₀, un groupe C(O)R₁₁ ou
un groupe phényle le cas échéant substitué avec une quelconque combinaison de un à trois membres choisis parmi le groupe comprenant un atome d'halogène, un groupe alkyle en C₁-C₆, un groupe halogénalkyle en C₁-C₆, un groupe alcoxy en C₁-C₆, un groupe halogénalcoxy en C₁-C₆, un groupe alkyl(en C₁-C₆)thio ou un groupe halogénalkyl(en C₁-C₆)thio, ou bien deux groupes R ou deux groupes R₄ adjacents peuvent être pris ensemble pour former un noyau, RR ou R₄R₄ étant représenté par : un groupe -OCH₂O-, un groupe -OCF₂O- ou un groupe -CH=CH-CH=CH- ;
R₆ et R₇ représentent chacun, indépendamment l'un de l'autre, un atome d'hydrogène, un groupe alkyle en C₁-C₆, un groupe halogénalkyle en C₁-C₆ ou
un groupe phényle le cas échéant substitué avec une quelconque combinaison de un à trois membres choisis parmi le groupe comprenant un atome d'halogène, un groupe alkyle en C₁-C₆, un groupe halogénalkyle en C₁-C₆, un groupe alcoxy en C₁-C₆, un groupe halogénalcoxy en C₁-C₄, un groupe alkyl(en C₁-C₆)thio ou un groupe halogénalkyl(en C₁-C₆)thio ;
R₈, R₉, R₁₃ et R₁₄ représentent chacun, indépendamment l'un de l'autre, un atome d'hydrogène, un groupe alkyle en C₁-C₆, un groupe alkyl(en C₁-C₆)carbonyle ou
un groupe phényle le cas échéant substitué avec une quelconque combinaison de un à trois membres choisis parmi le groupe comprenant un atome d'halogène, un groupe alkyle en C₁-C₆, un groupe halogénalkyle en C₁-C₆, un groupe alcoxy en C₁-C₆, un groupe halogénalcoxy en C₁-C₆, un groupe alkyl(en C₁-C₆)thio ou un groupe halogénalkyl(en C₁-C₆)thio ;
R₁₀ et R₁₁ représentent chacun, indépendamment l'un de l'autre, un atome d'hydrogène, un groupe alkyle en C₁-C₆ ou un groupe halogénalkyle en C₁-C₆ ;
R₁ et R₂ représentent chacun, indépendamment l'un de l'autre, un atome d'hydrogène, un groupe cycloalkyle en C₃-C₇, un groupe halogénalkyle en C₁-C₆, un groupe alcényle en C₃-C₆, un groupe halogénalcényle en C₃-C₆, un groupe alcynyle en C₃-C₆, un groupe halogénalcynyle en C₃-C₆, un groupe alcoxyalkyle en C₂-C₆, un groupe (CH₂)ᵥC(O)R₁₂,
un groupe alkyle en C₁-C₆ portant, le cas échéant, un substituant phénoxy ou phényle, le noyau phényle de chaque groupe étant substitué le cas échéant, de manière indépendante, avec de un à trois membres choisis parmi le groupe comprenant un atome d'halogène, un groupe alkyle en C₁-C₆, un groupe halogénalkyle en C₁-C₆, un groupe alcoxy en C₁-C₆, un groupe halogénalcoxy en C₁-C₆, un groupe alkyl(en C₁-C₆)thio ou un groupe halogénalkyl(en C₁-C₆)thio, un groupe phényle le cas échéant substitué avec de un à trois membres choisis parmi le groupe comprenant un atome d'halogène, un groupe alkyle en C₁-C₆, un groupe halogénalkyle en C₁-C₆, un groupe alcoxy en C₁-C₆, un groupe halogénalcoxy en C₁-C₆, un groupe alkyl(en C₁-C₆)thio ou un groupe halogénalkyl(en C₁-C₆)thio, ou
un noyau hétéroaromatique à 5 ou 6 membres, le cas échéant substitué avec une quelconque combinaison de un à trois membres choisis parmi le groupe comprenant un atome d'halogène, un groupe alkyle en C₁-C₆, un groupe halogénalkyle en C₁-C₆, un groupe alcoxy en C₁-C₆, un groupe halogénalcoxy en C₁-C₆, un groupe alkyl(en C₁-C₆)thio ou un groupe halogénalkyl(en C₁-C₆)thio, et
R₁ et R₂, lorsqu'ils sont pris ensemble avec l'atome auquel ils sont fixés, pouvant former un noyau cycloalkyle en C₃-C₆, R₁R₂ étant représenté par un groupe -(CH₂)ₜ dans lequel t représente 2, 3, 4 ou 5 ;
m, q et v représentent, chacun indépendamment l'un de l'autre, 0, 1 ou 2 ;
R₁₂ représente un atome d'hydrogène, un groupe alkyle en C₁-C₆, un groupe halogénalkyle en C₁-C₆, un groupe alcoxy en C₁-C₆, un groupe halogénalcoxy en C₁-C₆, un groupe alkyl(en C₁-C₆)thio, un groupe halogénalkyl(en C₁-C₆)thio ou un groupe NR₁₃R₁₄ ;
R₃ représente un atome d'hydrogène, un groupe alkyle en C₁-C₆, un groupe halogénalkyle en C₁-C₆ ou un groupe C(O)R₁₅ ;
R₁₅ représente un groupe alkyle en C₁-C₆, un groupe halogénalkyle en C₁-C₆, un groupe alcoxy en C₁-C₆ ou un groupe halogénalcoxy en C₁-C₆ ; et
R₅ représente un groupe alkyle en C₁-C₆,
un groupe phényle le cas échéant substitué avec une quelconque combinaison de un à trois membres choisis parmi le groupe comprenant un atome d'halogène, un groupe alkyle en C₁-C₆, un groupe halogénalkyle en C₁-C₆, un groupe alcoxy en C₁-C₆, un groupe halogénalcoxy en C₁-C₆, un groupe alkyl(en C₁-C₆)thio ou un groupe halogénalkyl(en C₁-C₆)thio, ou un groupe benzyle le cas échéant substitué sur le noyau phényle avec une quelconque combinaison de un à trois membres choisis parmi le groupe comprenant un atome d'halogène, un groupe alkyle en C₁-C₆, un groupe halogénalkyle en C₁-C₆, un groupe alcoxy en C₁-C₆, un groupe halogénalcoxy en C₁-C₆, un groupe alkyl(en C₁-C₆)thio ou un groupe halogénalkyl(en C₁-C₆)thio; et
leurs isomères optiques, ainsi que leurs sels acceptables du point de vue agricole.

2. Procédé selon la revendication 1, dans lequel le composé est choisi parmi le groupe constitué par
le 2-(*p*-chlorophényl)-5,5-diméthyl-4'-(trifluorométhoxy)-Δ²-1,3,4-oxadiazoline-4-carboxanilide ;
le 2-(*p*-chlorophényl)-5,5-diméthyl-4-(trifluorométhyl)-Δ²-1,3,4-oxadiazoline-4-carboxanilide ;
le 2-(*p*-bromophényl)-5,5-diméthyl-4'-(trifluorométhyl)-Δ²-1,3,4-oxadiazoline-4-carboxanilide ;
le 2-(*p*-fluorophényl)-5,5-diméthyl-4'-(trifluorométhyl)-Δ²-1,3,4-oxadiazoline-4-carboxanilide ;
le 5,5-diméthyl-2-(*p*-phénoxyphényl)-4'-[(trifluorométhyl)-thio]-Δ²-1,3,4-oxadiazoline-4-carboxanilide ;
le 2-(*p*-chlorophényl)-5-méthyl-4'-(trifluorométhoxy)-5-(trifluoro-méthyl)-Δ²-1,3,4-oxadiazoline-4-carboxanilide ;
le 5-(chlorométhyl)-2-(*p*-chlorophényl)-5-méthyl-4'-(trifluorométhyl)-Δ²-1,3,4-oxadiazoline-4-carboxanilide ;
le 4',5-bis(trifluorométhyl)-2-(*p*-fluorophényl)-5-méthyl-Δ²-1,3,4-oxadiazoline-4-carboxanilide ;
le 5-(chlorométhyl)-2-(*p*-fluorophényl)-5-méthyl-4'-(trifluorométhyl)-Δ²-1,3,4-oxadiazoline-4-carboxanilide ;
le 5-(chlorométhyl)-2-(*p*-fluorophényl)-5-méthyl-4'-(trifluoro-méthoxy)-Δ²-1,3,4-oxadiazoline-4-carboxanilide ;
le 2-(*p*-bromophényl)-5-(chlorométhyl)-5-méthyl-4'-(trifluorométhoxy)-Δ²-1,3,4-oxadiazoline-4-carboxanilide ;
le 2-(*p*-chlorophényl)-5-méthyl-5-(2,2,2-trifluoroéthyl)-4'-(trifluorométhyl)-Δ²-1,3,4-oxadiazoline-4-carboxanilide ;
le 2-(*p*-chlorophényl)-5-méthyl-5-(2,2,2-trifluoroéthyl)-4'-(trifluorométhoxy)-Δ²-1,3,4-oxadiazoline-4-carboxanilide ;
le 2-(*p*-chlorophényl)-5-méthyl-5-(2-pyridyl)-4'-(trifluorométhyl)-Δ²-1,3,4-oxadiazoline-4-carboxanilide ;
le 2-(*p*-chlorophényl)-5-méthyl-5-(2-pyridyl)-4'-(trifluorométhoxy)-Δ²-1,3,4-oxadiazoline-4-carboxanilide ;
le N-{[2-(*p*-chlorophényl)-5,5-diméthyl-Δ²-1,3,4-oxadiazolin-4-yl]-carbonyl}-*p*-(trifluorométhoxy)-carbanilate de méthyle ;
le N-{[2-(*p*-chlorophényl)-5,5-diméthyl-Δ²-1,3,4-oxadiazolin-4-yl]-carbonyl}-*p*-(trifluorométhyl)-carbanilate de méthyle ; et
le 2-(*p*-chlorophényl)-5-méthyl-4-{[*p*-(trifluorométhoxy)phényl]-carbamoyl}-Δ²-1,3,4-oxadiazoline-5-acétate de méthyle.

3. Procédé pour protéger des plantes en cours de croissance contre une attaque ou une infestation par des organismes nuisibles, à savoir des insectes ou des acariens, qui comprend le fait d'appliquer au feuillage des plantes ou encore au sol ou à l'eau dans lequel ou dans laquelle elles croissent, une quantité efficace du point de vue pesticide d'un composé répondant à la formule développée 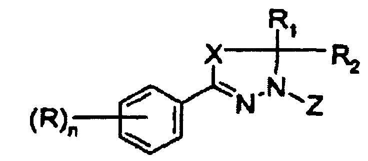 dans laquelle n, R, R₁, R₂, X et Z sont tels que décrits à la revendication 1.

4. Procédé selon la revendication 3, dans lequel le composé est choisi parmi le groupe constitué par
le 2-(*p*-chlorophényl)-5,5-diméthyl-4'-(trifluorométhoxy)-Δ²-1,3,4-oxadiazoline-4-carboxanilide ;
le 2-(*p*-chlorophényl)-5,5-diméthyl-4'-(trifluorométhyl)-Δ²-1,3,4-oxadiazoline-4-carboxanilide ;
le 2-(*p*-bromophényl)-5,5-diméthyl-4'-(trifluorométhyl)-Δ²-1,3,4-oxadiazoline-4-carboxanilide ;
le 2-(*p*-fluorophényl)-5,5-diméthyl-4'-(trifluorométhyl)-Δ²-1,3,4-oxadiazoline-4-carboxanilide ;
le 5,5-diméthyl-2-(*p*-phénoxyphényl)-4'-[(trifluorométhyl)-thio]-Δ²-1,3,4-oxadiazoline-4-carboxanilide ;
le 2-(*p*-chlorophényl)-5-méthyl-4'-(trifluorométhoxy)-5-(trifluoro-méthyl)-Δ²-1,3,4-oxadiazoline-4-carboxanilide ;
le 5-(chlorométhyl)-2-(*p*-chlorophényl)-5-méthyl-4'-(trifluorométhyl)-Δ²-1,3,4-oxadiazoline-4-carboxanilide ;
le 4',5-bis(trifluorométhyl)-2-(*p*-fluorophényl)-5-méthyl-Δ²-1,3,4-oxadiazoline-4-carboxanilide ;
le 5-(chlorométhyl)-2-(*p*-fluorophényl)-5-méthyl-4'-(trifluorométhyl)-Δ²-1,3,4-oxadiazoline-4-carboxanilide ;
le 5-(chlorométhyl)-2-(*p*-fluorophényl)-5-méthyl-4'-(trifluoro-méthoxy)-Δ²-1,3,4-oxadiazoline-4-carboxanilide ;
le 2-(*p*-bromophényl)-5-(chlorométhyl)-5-méthyl-4'-(trifluorométhoxy)-Δ²-1,3,4-oxadiazoline-4-carboxanilide ;
le 2-(*p*-chlorophényl)-5-méthyl-5-(2,2,2-trifluoroéthyl)-4'-(trifluorométhyl)-Δ²-1,3,4-oxadiazoline-4-carboxanilide ;
le 2-(*p*-chlorophényl)-5-méthyl-5-(2,2,2-trifluoroéthyl)-4'-(trifluorométhoxy)-Δ²-1,3,4-oxadiazoline-4-carboxanilide ;
le 2-(*p*-chlorophényl)-5-méthyl-5-(2-pyridyl)-4'-(trifluorométhyl)-Δ²-1,3,4-oxadiazoline-4-carboxanilide ;
le 2-(*p*-chlorophényl)-5-méthyl-5-(2-pyridyl)-4'-(trifluorométhoxy)-Δ²-1,3,4-oxadiazoline-4-carboxanilide ;
le N-{[2-(*p*-chlorophényl)-5,5-diméthyl-Δ²-1,3,4-oxadiazolin-4-yl]-carbonyl}-*p*-(trifluorométhoxy)-carbanilate de méthyle ;
le N-{[2-(*p*-chlorophényl)-5,5-diméthyl-Δ²-1,3,4-oxadiazolin-4-yl]-carbonyl}-*p*-(trifluorométhyl)-carbanilate de méthyle ; et
le 2-(*p*-chlorophényl)-5-méthyl-4-{[*p*-(trifluorométhoxy)phényl]-carbamoyl}-Δ²-1,3,4-oxadiazoline-5-acétate de méthyle.

5. Procédé selon la revendication 3, dans lequel le composé est appliqué sur les plantes, ou bien sur le sol ou sur l'eau dans lequel ou dans laquelle elles croissent, à raison de 0,1 kg/ha à 4,0 kg/ha.

6. Composé répondant à la formule développée II 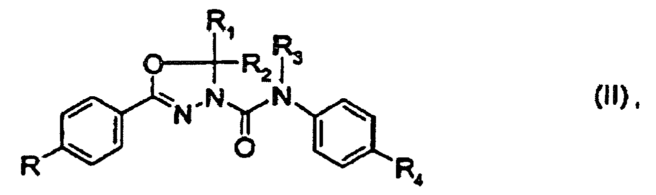 dans laquelle
R représente un atome d'halogène, un groupe halogénalkyle en C₁-C₄, un groupe halogénalcoxy en C₁-C₄ ou un groupe phénoxy, le cas échéant substitué avec une quelconque combinaison de un à trois membres choisis parmi le groupe comprenant un atome d'halogène, un groupe alkyle en C₁-C₄, un groupe halogénalkyle en C₁-C₄, un groupe alcoxy en C₁-C₄ ou un groupe halogénalcoxy en C₁-C₄ ;
R₄ représente un groupe halogénalkyle en C₁-C₄, un groupe halogénalcoxy en C₁-C₄ ou un groupe halogénalkyl(en C₁-C₄)thio ;
R₁ représente un groupe alkyle en C₁-C₄ ;
R₂ représente un groupe alkyle en C₁-C₄, un groupe halogénalkyle en C₁-C₄, un groupe (CH₂)ᵥC(O)R₁₂ ou un groupe 2-pyridyle le cas échéant substitué avec une quelconque combinaison de un à trois membres choisis parmi le groupe comprenant un atome d'halogène, un groupe alkyle en C₁-C₄, un groupe halogénalkyle en C₁-C₄, un groupe alcoxy en C₁-C₄ ou un groupe halogénalcoxy en C₁-C₄ ;
v représente 0 ou 1 ;
R₁₂ représente un groupe alcoxy en C₁-C₄ ou un groupe halogénalcoxy en C₁-C₄ ;
R₃ représente un atome d'hydrogène ou un groupe C(O)R₁₅ ; et
R₁₅ représente un groupe alcoxy en C₁-C₄.

7. Composé choisi parmi le groupe constitué par
le 2-(*p*-chlorophényl)-5,5-diméthyl-4'-(trifluorométhoxy)-Δ²-1,3,4-oxadiazoline-4-carboxanilide ;
le 2-(*p*-chlorophényl)-5,5-diméthy)-4'-(trifluorométhyl)-Δ²-1,3,4-oxadiazoline-4-carboxanilide ;
le 2-(*p*-bromophényl)-5,5-diméthyl-4'-(trifluorométhyl)-Δ²-1,3,4-oxadiazoline-4-carboxanilide ;
le 2-(*p*-fluorophényl)-5,5-diméthyl-4'-(trifluorométhyl)-Δ²-1,3,4-oxadiazoline-4-carboxanilide ;
le 5,5-diméthyl-2-(*p*-phénoxyphényl)-4'-[(trifluorométhyl)-thio]-Δ²-1,3,4-oxadiazoline-4-carboxanilide ;
le 2-(*p*-chlorophényl)-5-méthyl-4'-(trifluorométhoxy)-5-(trifluoro-méthyl)-Δ²-1,3,4-oxadiazoline-4-carboxanilide ;
le 5-(chlorométhyl)-2-(*p*-chlorophényl)-5-méthyl-4'-(trifluorométhyl)-Δ²-1,3,4-oxadiazoline-4-carboxanilide ;
le 4',5-bis(trifluorométhyl)-2-(*p*-fluorophényl)-5-méthyl-Δ²-1,3,4-oxadiazoline-4-carboxanilide ;
le 5-(chlorométhyl)-2-(*p*-fluorophényl)-5-méthyl-4'-(trifluorométhyl)-Δ²-1,3,4-oxadiazoline-4-carboxanilide ;
le 5-(chlorométhyl)-2-(*p*-fluorophényl)-5-méthyl-4'-(trifluoro-méthoxy)-Δ²-1,3,4-oxadiazoline-4-carboxanilide ;
le 2-(*p*-bromophényl)-5-(chlorométhyl)-5-méthyl-4'-(trifluorométhoxy)-Δ²-1,3,4-oxadiazoline-4-carboxanilide ;
le 2-(*p*-chlorophényl)-5-méthyl-5-(2,2,2-trifluoroéthyl)-4'-(trifluorométhyl)-Δ²-1,3,4-oxadiazoline-4-carboxanilide ;
le 2-(*p*-chlorophényl)-5-méthyl-5-(2,2,2-trifluoroéthyl)-4'-(trifluorométhoxy)-Δ²-1,3,4-oxadiazoline-4-carboxanilide ;
le 2-(*p*-chlorophényl)-5-méthyl-5-(2-pyridyl)-4'-(trifluorométhyl)-Δ²-1,3,4-oxadiazoline-4-carboxanilide ;
le 2-(*p*-chlorophényl)-5-méthyl-5-(2-pyridyl)-4'-(trifluorométhoxy)-Δ²-1,3,4-oxadiazoline-4-carboxanilide ;
le N-{[2-(*p*-chlorophényl)-5,5-diméthyl-Δ²-1,3,4-oxadiazolin-4-yl]-carbonyl}-*p*-(trifluorométhoxy)-carbanilate de méthyle ;
le N-{[2-(*p*-chlorophényl)-5,5-diméthyl-Δ²-1,3,4-oxadiazolin-4-yl]-carbonyl}-*p*-(trifluorométhyl)-carbanilate de méthyle ; et
le 2-(*p*-chlorophényl)-5-méthyl-4-{[*p*-(trifluorométhoxy)phényl]-carbamoyl}-Δ²-1,3,4-oxadiazoline-5-acétate de méthyle.

8. Composition pour lutter contre des organismes nuisibles, à savoir des insectes ou des acariens, qui comprend un support acceptable du point de vue agronomique et une quantité efficace du point de vue pesticide d'un composé répondant à la formule développée II 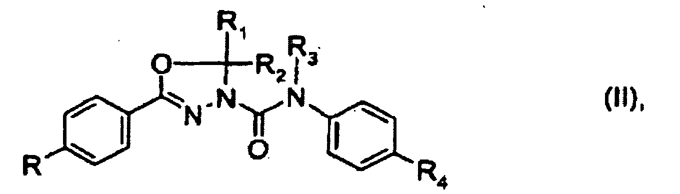 dans laquelle
R représente un atome d'halogène, un groupe halogénalkyle en C₁-C₄, un groupe halogénalcoxy en C₁-C₄ ou un groupe phénoxy, le cas échéant substitué avec une quelconque combinaison de un à trois membres choisis parmi le groupe comprenant un atome d'halogène, un groupe alkyle en C₁-C₄, un groupe halogénalkyle en C₁-C₄, un groupe alcoxy en C₁-C₄ ou un groupe halogénalcoxy en C₁-C₄ ;
R₄ représente un groupe halogénalkyle en C₁-C₄, un groupe halogénalcoxy en C₁-C₄ ou un groupe halogénalkyl(en C₁-C₄)thio ;
R₁ représente un groupe alkyle en C₁-C₄ ;
R₂ représente un groupe alkyle en C₁-C₄, un groupe halogénalkyle en C₁-C₄, un groupe (CH₂)ᵥC(O)R₁₂ ou un groupe 2-pyridyle le cas échéant substitué avec une quelconque combinaison de un à trois membres choisis parmi le groupe comprenant un atome d'halogène, un groupe alkyle en C₁-C₄, un groupe halogénalkyle en C₁-C₄, un groupe alcoxy en C₁-C₄ ou un groupe halogénalcoxy en C₁-C₄ ;
v représente 0 ou 1 ;
R₁₂ représente un groupe alcoxy en C₁-C₄ ou un groupe halogénalcoxy en C₁-C₄ ;
R₃ représente un atome d'hydrogène ou un groupe C(O)R₁₅ ; et
R₁₅ représente un groupe alcoxy en C₁-C₄.

9. Composition selon la revendication 8, dans laquelle le composé est choisi parmi le groupe constitué par
le 2-(*p*-chlorophényl)-5,5-diméthyl-4'-(trifluorométhoxy)-Δ²-1,3,4-oxadiazoline-4-carboxanilide ;
le 2-(*p*-chlorophényl)-5,5-diméthyl-4'-(trifluorométhyl)-Δ²-1,3,4-oxadiazoline-4-carboxanilide ;
le 2-(*p*-bromophényl)-5,5-diméthyl-4'-(trifluorométhyl)-Δ²-1,3,4-oxadiazoline-4-carboxanilide ;
le 2-(*p*-fluorophényl)-5,5-diméthyl-4'-(trifluorométhyl)-Δ²-1,3,4-oxadiazoline-4-carboxanilide ;
le 5,5-diméthyl-2-(*p*-phénoxyphényl)-4'-[(trifluorométhyl)-thio]-Δ²-1,3,4-oxadiazoline-4-carboxanilide ;
le 2-(*p*-chlorophényl)-5-méthyl-4'-(trifluorométhoxy)-5-(trifluoro-méthyl)-Δ²-1,3,4-oxadiazoline-4-carboxanilide ;
le 5-(chlorométhyl)-2-(*p*-chlorophényl)-5-méthyl-4'-(trifluorométhyl)-Δ²-1,3,4-oxadiazoline-4-carboxanilide ;
le 4',5-bis(trifluorométhyl)-2-(*p*-fluorophényl)-5-méthyl-Δ²-1,3,4-oxadiazoline-4-carboxanilide ;
le 5-(chlorométhyl)-2-(*p*-fluorophényl)-5-méthyl-4'-(trifluorométhyl)-Δ²-1,3,4-oxadiazoline-4-carboxanilide ;
le 5-(chlorométhyl)-2-(*p*-fluorophényl)-5-méthyl-4'-(trifluoro-méthoxy)-Δ²-1,3,4-oxadiazoline-4-carboxanilide ;
le 2-(*p*-bromophényl)-5-(chlorométhyl)-5-méthyl-4'-(trifluorométhoxy)-Δ²-1,3,4-oxadiazoline-4-carboxanilide ;
le 2-(*p*-chlorophényl)-5-méthyl-5-(2,2,2-trifluoroéthyl)-4'-(trifluorométhyl)-Δ²-1,3,4-oxadiazoline-4-carboxanilide ;
le 2-(*p*-chlorophényl)-5-méthyl-5-(2,2,2-trifluoroéthyl)-4'-(trifluorométhoxy)-Δ²-1,3,4-oxadiazoline-4-carboxanilide ;
le 2-(*p*-chlorophényl)-5-méthyl-5-(2-pyridyl)-4'-(trifluorométhyl)-Δ²-1,3,4-oxadiazoline-4-carboxanilide ;
le 2-(*p*-chlorophényl)-5-méthyl-5-(2-pyridyl)-4'-(trifluorométhoxy)-Δ²-1,3,4-oxadiazoline-4-carboxanilide ;
le N-{[2-(*p*-chlorophényl)-5,5-diméthyl-Δ²-1,3,4-oxadiazolin-4-yl]-carbonyl}-*p*-(trifluorométhoxy)-carbanilate de méthyle ;
le N-{[2-(*p*-chlorophényl)-5,5-diméthyl-Δ²-1,3,4-oxadiazolin-4-yl]-carbonyl}-*p*-(trifluorométhyl)-carbanilate de méthyle ;
le 2-(*p*-chlorophényl)-5-méthyl-4-{[*p*-(trifluorométhoxy)phényl]-carbamoyl}-Δ²-1,3,4-oxadiazoline-5-acétate de méthyle.
